(19) Europäisches Patentamt
European Patent Office
Office européen des brevets

(11) **EP 3 256 142 B1**

(12) **EUROPÄISCHE PATENTSCHRIFT**

(45) Veröffentlichungstag und Bekanntmachung des Hinweises auf die Patenterteilung:
**06.11.2019  Patentblatt 2019/45**

(21) Anmeldenummer: **16704186.2**

(22) Anmeldetag: **10.02.2016**

(51) Int Cl.:
*A61K 36/85* (2006.01)     *A61K 36/886* (2006.01)
*A61K 36/28* (2006.01)     *A61K 36/32* (2006.01)
*A61K 36/68* (2006.01)     *A61P 17/10* (2006.01)
*A61P 31/00* (2006.01)

(86) Internationale Anmeldenummer:
**PCT/EP2016/052836**

(87) Internationale Veröffentlichungsnummer:
**WO 2016/128471 (18.08.2016 Gazette 2016/33)**

(54) **IMMUNOLOGISCH AKTIVES PHYTO-GEMISCH UND SEINE ANWENDUNG BEI DER PRÄVENTION UND IN EINEM VERFAHREN ZUR BEHANDLUNG VON EFFLORESZENZEN**

IMMUNOLOGICALLY ACTIVE PLANT MIXTURE AND USE THEREOF FOR PROPHYLAXIS AND IN A METHOD FOR TREATING EFFLORESCENCE

MÉLANGE PHYTOTHÉRAPEUTIQUE IMMUNOLOGIQUEMENT ACTIF ET APPLICATION DUDIT MÉLANGE POUR LA PRÉVENTION ET DANS UN PROCÉDÉ DESTINÉ AU TRAITEMENT DES EFFLORESCENCES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.02.2015  DE 102015102020**

(43) Veröffentlichungstag der Anmeldung:
**20.12.2017  Patentblatt 2017/51**

(73) Patentinhaber: **Wilmanowicz, Renate**
**40227 Düsseldorf (DE)**

(72) Erfinder: **Wilmanowicz, Renate**
**40227 Düsseldorf (DE)**

(74) Vertreter: **Heide, Anna Katharina**
**Ruhr-IP Patentanwälte**
**Brucker Holt 58**
**45133 Essen (DE)**

(56) Entgegenhaltungen:
• **JAMES B. HUDSON: "Applications of the Phytomedicine Echinacea purpurea (Purple Coneflower) in Infectious Diseases", JOURNAL OF BIOMEDICINE AND BIOTECHNOLOGY, Bd. 8, Nr. 5, 1. Januar 2012 (2012-01-01), Seiten 401-16, XP055263118, US ISSN: 1110-7243, DOI: 10.1300/J157v05n02_04**
• **ROJAS JHON J ET AL: "Screening for antimicrobial activity of ten medicinal plants used in Colombian folkloric medicine: A possible alternative in the treatment of non-nosocomial infections", BMC COMPLEMENTARY AND ALTERNATIVE MEDICINE, BIOMED CENTRAL LTD., LONDON, GB, Bd. 6, Nr. 1, 17. Februar 2006 (2006-02-17), Seite 2, XP021017341, ISSN: 1472-6882, DOI: 10.1186/1472-6882-6-2**
• **MANTARING-CHUA NIMFA: "Quantitative microbial assay, clinical testing and stability studies of the crude leaf extract of Bidens pilosa LINN", ACTA MANILANA. SERIES A, NATURAL AND APPLIED SCIENCES, MANILA, PH, Bd. 39, 1. Januar 1991 (1991-01-01), Seiten 31-37, XP009189308, ISSN: 0065-1370**
• **DATABASE WPI Week 200167 Thomson Scientific, London, GB; AN 2001-592583 XP002756143, & JP 2001 178390 A (MUSASHINO MENEKI KENKYUSHO KK) 3. Juli 2001 (2001-07-03)**

- **CHARIANDY C M ET AL: "Screening of medicinal plants from Trinidad and Tobago for antimicrobial and insecticidal properties", JOURNAL OF ETHNOPHARMACOLOGY, ELSEVIER IRELAND LTD, IE, Bd. 64, Nr. 3, 21. Juli 1998 (1998-07-21), Seiten 265-270, XP028142962, ISSN: 0378-8741, DOI: 10.1016/S0378-8741(98)00130-5 [gefunden am 2011-02-15]**
- **Okoye Tc Akah Pa Okoli Co Ezike Ac Mbaoji Fn ET AL: "Antimicrobial and antispasmodic activity of leaf extract and fractions of Stachytarpheta cayennensis", &#x4E9A;&#x592A;&#x70ED;&#x5E26;&#x533B;&# x836F;?&#x5FD7;&#xFF1A;&#x82F1;??, 1. Januar 2010 (2010-01-01), Seiten 189-192, XP055261695, DOI: 10.1016/S1995-7645(10)60006-5 Gefunden im Internet: URL:http://ac.els-cdn.com/S199576451060006 5/1-s2.0-S1995764510600065-main.pdf?_tid=9 bb3b9aa-f718-11e5-a0d6-00000aab0f01&acdnat =1459412256_a367dc7fb3a73e9177b3aaf56b414 d 94**
- **A CAMPORESE ET AL: "Screening of anti-bacterial activity of medicinal plants from Belize (Central America)", JOURNAL OF ETHNOPHARMACOLOGY, Bd. 87, Nr. 1, 1. Juli 2003 (2003-07-01), Seiten 103-107, XP055006356, ISSN: 0378-8741, DOI: 10.1016/S0378-8741(03)00115-6**
- **ELSTON D M: "Topical Antibiotics in Dermatology: Emerging Patterns of Resistance", DERMATOLOGIC CLINICS, W.B. SAUNDERS CO., LONDON, GB, Bd. 27, Nr. 1, 1. Januar 2009 (2009-01-01) , Seiten 25-31, XP008143934, ISSN: 0733-8635, DOI: 10.1016/J.DET.2008.07.004**
- **FLOYD A RUSSELL ET AL: "Stemodin-derived analogues with lipid peroxidation, cyclooxygenase enzymes and human tumour cell proliferation inhibitory activities", PHYTOCHEMISTRY, Bd. 72, Nr. 18, 21. September 2011 (2011-09-21), Seiten 2361-2368, XP028323411, ISSN: 0031-9422, DOI: 10.1016/J.PHYTOCHEM.2011.08.024 [gefunden am 2011-08-25]**

**Beschreibung**

Die vorliegende Erfindung betrifft ein immunologisch aktives Phyto-Gemisch umfassend mindestens einen ethanolischen Pflanzenextrakt ausgewählt aus a) Bidens alba, Bidens pilosa aus dem Genus Bidens der Familie Asteraceae und mindestens einen weiteren ethanolischen Pflanzenextrakt ausgewählt aus b) Stachytarpheta jamaicensis, Stachytarpheta cayennensis, Stachytarpheta indica aus dem Genus Stachytarpheta der Familie Verbenaceae und/oder c) Bursera microphylla, Bursera glabrifolia und Bursera simaruba aus dem Genus Bursera der Familie Burseraceae. Das Phyto-Gemisch enthält optional d) mindestens einen weiteren biologisch aktiven Pflanzenextrakt, wie Aloe vera und/oder Stemodia maritima. Das vorgenannte erfindungsgemäße Phyto-Gemisch weist eine gute antimikrobielle gegen eine transiente Hautflora und anti-entzündliche Wirksamkeit auf und ist besonders zur Prävention und Behandlung von Effloreszenzen geeignet. Daher betrifft die vorliegende Erfindung ebenfalls eine Zubereitung zur oralen und topischen Verabreichung zur Prävention und Behandlung von Effloreszenzen der Haut und Schleimhaut.

In der heutigen Medizin werden den Patienten umfassende synthetisch hergestellte pharmazeutische Erzeugnisse zur Prävention oder Behandlung von Hautirritationen und Hauterkrankungen bereitgestellt. Diese werden als zugelassene Arzneimittel durch den Arzt verschrieben oder stehen als rezeptfreie Medizinprodukte zur Verfügung. Allerdings rufen die pharmazeutischen Zusammensetzungen durch die synthetischen Wirkstoffe vermehrt Nebenwirkungen auf, lösen allergische Reaktionen aus oder führen zu stetig zunehmenden Resistenzen bei den die Hautirritation oder -erkrankung auslösenden Infektionskeimen.

Daher gibt es immer mehr Bestrebungen neue Wirkstoffe zu isolieren, die gegen resistente Keime weiterhin eine Wirksamkeit aufweisen, bei Menschen weniger oder keine Nebenwirkungen oder allergische Reaktionen auslösen und potentiell eine verbesserte Resorption beim Menschen aufweisen. Als Quellen neuer Wirkstoffe dienen hierzu Mikroorganismen selbst oder Pflanzen.

Bei pflanzlichen Quellen werden einzelne Verbindungsklassen, wie z.B. Flavone isoliert und in pharmazeutische Zubereitungen verarbeitet. Solche Flavonextrakte offenbart die RU2412719 für Arzneimittel zur Behandlung von Lebererkrankungen. Die CN102743651 offenbart eine Mischung aus 30 verschiedenen Pflanzen in Form einer Lotion zur Behandlung von Cellulitis.

Im Stand der Technik werden diverse Pflanzen, insbesondere aus der traditionellen Medizin auf Ihre Wirksamkeit getestet, ihre Inhaltsstoffe identifiziert, die Toxizität untersucht, aber nur geringe oder keine Wirksamkeiten festgestellt (EP114709A1).

ROJAS JHON J et. al. ("Screening for antimicrobial activity of ten medicinal plants used in Colombian folkloric medicine: A possible alternative in the treatment of non-nosocomial infections", BMC COMPLEMENTARY AND ALTERNATIVE MEDICINE, BIOMED CENTRAL LTD., LONDON, GB, Bd. 6, Nr. 1, 17. Februar 2006 (2006-02-17), Seite 2, XP021017341, ISSN: 1472-6882, DOI: 10.1186/1472-6882-6-2) offenbart die antimikrobielle Aktivität von Extrakten aus *Bidens pilosa* gegenüber verschiedenen Testkeimen, sowie deren Verwendung zur Behandlung von Infektionen.

MANTARING-CHUA NIMFA ("Quantitative microbial assay, clinical testing and stability studies of the crude leaf extract of Bidens pilosa LINN", ACTA MAN1LANA. SERIES A, NATURAL AND APPLIED SCIENCES, MANILA, PH, Bd. 39, 1. Januar 1991 (1991-01-01), Seiten 31-37, XP009189308, ISSN: 0065-1370) offenbart die antimikrobielle Aktivität von Extrakten aus *Bidens pilosa* gegenüber verschiedenen Testkeimen, sowie deren pH-Stabilität.

JP 2001 178390 A (MUSASHINO MENEKI KENKYUSHO KK) offenbart die antibakterielle und antientzündliche Aktivität von Extrakten aus *Bidens pilosa* gegenüber verschiedenen Testkeimen, sowie deren Verwendung in Arznei- und Nahrungsmitteln.

CHARIANDY C M et. al. ("Screening of medicinal plants from Trinidad and Tobago for antimicrobial and insecticidal properties", JOURNAL OF ETHNOPHARMACOLOGY, ELSEVIER IRELAND LTD, IE, Bd. 64, Nr. 3, 21. Juli 1998 (1998-07-21), Seiten 265-270, XP028142962, ISSN: 0378-8741, DOI: 10.1016/80378-8741(98)00130-5) offenbart die antimikrobielle Aktivität von Extrakten aus *Bidens pilosa* sowie aus *Stachytarpheta jamaicenis*.

Okoye Tc et al. ("Antimicrobial and antispasmodic activity of leaf extract and fractions of Stachytarpheta cayennensis", 1. Januar 2010 (2010-01-01), Seiten 189-192, XP055261695, DOI: 10.1016/S1995-7645(10)60006-5) beschreibt die antimikrobielle Aktivität von *Stachytarpheta cayennensis*.

Die antibakterielle Eigenschaften von *Bursera simaruba* werden in Camporese A. et al. ("Screening of antibacterial activity of medicinal plants from Belize (Central America)", JOURNAL OF ETHNOPHARMACOLOGY, Bd. 87, Nr. 1, 1. Juli 2003 (2003-07-01), Seiten 103-107, XP055006356, ISSN: 0378-8741, DOI; 10.1016/80378-8741(03)00115-6) und in Elston D. M. ("Topical Antibiotics in Dermatology: Emerging Patterns of Resistance", DERMATOLOGIC CLINICS, W.B. SAUNDERS CO., LONDON, GB, Bd. 27, Nr. 1, 1. Januar 2009 (2009-01-01), Seiten 25-31, XP008143934, ISSN: 0733-8635, DOI: 10.1016/J.DET.2008.07.004) beschrieben.

Nur sehr wenige Untersuchungen wurden bislang zu pflanzlichen Zubereitungen durchgeführt und keine weist sowohl eine antibakterielle als auch anti-entzündliche Wirkung nach. Somit wurde bislang keine pflanzliche Alternative zu synthetischen Wirkstoffen, wie z.B. Antibiotika oder synthetischen Entzündungshemmern, die beide Wirksamkeiten aufweisen, nachgewiesen.

Die vorliegende Erfindung stellt sich die Aufgabe neue Zubereitungen mit wirksamen Bestandteilen aus natürlichen Quellen, insbesondere wirksamen Bestandteilen aus pflanzlichen Quellen, bereitzustellen. Eine weitere Aufgabe besteht darin ein pflanzliches Erzeugnis und Zubereitungen mit antimikrobiell, vorzugsweise antibakteriell, und/oder anti-entzündlich wirksamen Bestandteilen bereitzustellen. Dabei sollen unterschiedliche mögliche Kombinationen einzelner pflanzlicher Erzeugnisse, wie Pflanzenextrakte bereitgestellt werden, die individuell für den Anwender gemischt werden können. Eine weitere Aufgabe ist die Bereitstellung eines Phyto-Gemisches zur Herstellung von Zubereitungen zur präventiven Anwendung oder therapeutischen Anwendung bei der Behandlung von Effloreszenzen. Dazu sollen pharmazeutische Zusammensetzungen, Pflegeprodukte, Nahrungsergänzungsmittel sowie Medizinprodukte enthaltend ein Phyto-Gemisch aus Totalextrakten von Pflanzen bereitgestellt werden. Weiterhin stellt sich die vorliegende Erfindung die Aufgabe ein natürliches Produkt als Alternative zu synthetisch hergestellten Produkten bereitzustellen. Es besteht die Aufgabe ein natürliches Produkt aus pflanzlichen Quellen insbesondere für Menschen bereitzustellen, bei denen die aus dem Stand der Technik bekannten synthetisch hergestellten Produkte eine stark reduzierte oder keine Wirksamkeit mehr aufweisen und/oder vermehrt Nebenwirkungen hervorrufen. Die vorgenannten pflanzlichen Erzeugnisse und seine Zubereitungen enthaltend das Phyto-Gemisch sollen zur Prävention und Behandlung von Effloreszenzen bereitgestellt werden.

Die vorliegende Erfindung stellt daher als ein natürliches Produkt aus pflanzlichen Quellen ein Phyto-Gemisch bereit, das sowohl antimikrobiell, vorzugsweise antibakteriell, als auch anti-entzündlich wirkt.

Somit ist ein Gegenstand der vorliegenden Erfindung ein immunologisch aktives Phyto-Gemisch, welches eine antimikrobielle Wirksamkeit gegen eine transiente Hautflora und eine anti-entzündliche Wirksamkeit aufweist, vorzugsweise antibakterielle und/oder antimykotische Wirksamkeit, wobei das Phyto-Gemisch mindestens einen ethanolischen Pflanzenextrakt ausgewählt aus

a) Bidens alba (Bid. alba), Bidens pilosa (Bid. pilosa), aus dem Genus Bidens der Familie Asteraceae, und mindestens einen weiteren ethanolischen Pflanzenextrakt umfasst, ausgewählt aus

b) Stachytarpheta jamaicensis (Sta. jamaicensis), Stachytarpheta indica (Sta. indica) und Stachytarpheta cayennensis (Sta. cayennensis) aus dem Genus Stachytarpheta der Familie Verbenaceae und/oder

c) Bursera microphylla (Bur. microphylla), Bursera. glabrifolia (Bur. glabrifolia und Bursera simaruba (Bur. simaruba) aus dem Genus Bursera der Familie Burseraceae und optional zusätzlich d) mindestens einen weiteren Extrakt einer biologisch aktiven Pflanze, wie nachfolgend beschrieben.

Wenn die Formulierung "Pflanzenextrakte a) und b) und/oder c)" ohne Einschränkung auf eine konkrete Spezies verwendet wird, so ist dies als Abkürzung der vorstehenden Formulierung zu verstehen. Nachfolgend wird die Bezeichnung des jeweiligen Genus a) Bidens mit "Bid.", b) Stachytarpheta mit "Sta." und c) Bursera mit "Bur." abgekürzt.

Die vorgenannten Pflanzenextrakte sind ethanolische Extrakte (größer gleich 70 % Ethanol, Rest Wasser). Wässrige Extrakte werden beschrieben.

Das erfindungsgemäße "immunologisch aktive Phyto-Gemisch" wird kurz, also synonym, als "Phyto-Gemisch" bezeichnet.

"Immunologisch aktiv" im Sinne der Erfindung bedeutet, dass das erfindungsgemäße Phyto-Gemisch, insbesondere die enthaltenden Inhaltsstoffe und Verbindungen, Immunreaktionen des Körpers verhindert, hemmt oder reduziert. "Immunologisch aktiv" umfasst eine präventive Wirkung, sodass Immunreaktionen des Körpers erst gar nicht ausgelöst werden, und therapeutisch, sodass die bereits im Körper ausgelöste Immunreaktion gestoppt, herabreguliert oder vermindert wird. Abhängig vom Verabreichungszeitpunkt, kann die beginnende Immunreaktion gehemmt werden bevor die mit der Immunreaktion einhergehenden Symptome und phänotypischen Erscheinungen auftreten. Auslöser der vorgenannten Immunreaktionen können sein körpereigene Mikroorganismen, in/auf den Körper einwirkende Erreger, wie Bakterien, Viren, Pilze und Parasiten, oder toxisch und/der Allergiereaktionen auslösende, einwirkende Verbindungen.

Als Immunreaktionen wird die Immunantwort des Immunsystems auf einen Organismus, insbesondere auf Mikroorganismen oder Substanzen, insbesondere Toxine, bezeichnet.

Was als Auslöser der vorgenannten Immunreaktion dient, hängt von der Konstitution des Organismus, Menschen oder Tieres, ab. Somit muss differenziert werden zwischen einem gesunden Organismus und einem immunsuppressiven Organismus.

Ein gesunder Organismus, insbesondere Mensch, weist keine angeborene Erkrankung, insbesondere keine immunschwächende Erkrankung, auf und verfügt somit über ein normal funktionierendes und reagierendes Immunsystem.

Ein immungeschwächter Organismus, insbesondere immungeschwächter Mensch, weist ein geschwächtes Immunsystem auf. Die Schwächung kann durch eine temporäre Erkrankung, wie Grippe oder Erkältung, eine lang andauernde Erkrankung, wie Krebs, Mangel-/ Unterernährung, Infektionen mit bestimmten Erregern, sowie Einnahmen bestimmter Medikamente, z.B. Chemotherapeutika, oder Strahlung erfolgen. Die vorgenannte Schwächung ist eine erworbene Immundefizienz, die von einer angeborenen Immundefizienz zu unterscheiden ist. Eine angeborene Immundefizienz basiert auf Mutationen in Genen, die z.B. die Produktion oder Funktion von Antikörpern oder Phagozyten beeinträchtigen, die T-Zell- oder B-Zell-dominierte Immunantworten betreffen.

Die vorliegende Erfindung betrifft vorzugsweise ein immunologisch aktives Phyto-Gemisch, das präventiv oder zur Behandlung von Effloreszenzen bei gesunden Menschen und erworben immungeschwächten Menschen geeignet ist.

In einer weiteren Ausführungsform umfasst das erfindungsgemäße immunologisch aktive Phyto-Gemisch, optional zusätzlich d) mindestens einen weiteren Extrakt einer biologisch aktiven Pflanze umfassend Aloe-Spezies des Genus Aloe der Unterfamilie Asphodeloideae, Spezies des Genus Stemodia (kurz "Stem.") der Familie Plantaginaceae und Stem. maritima. Bevorzugte Aloe-Spezies sind Aloe vera, Aloe barbadensis, Aloe perfoliata, Aloe vulgaris, Aloe indica und Aloe chinensis.

Die Familie Asteraceae umfasst den a) Genus Bidens (=A), der diverse Bidens-Spezies umfasst, die umgangssprachlich auch als "Beggartick" beschrieben werden. Der Genus Bidens wurde vormals vom Fachmann der Familie Compositae zugeordnet. Der Genus Bidens umfasst die Spezies Bid. alba, Bid. pilosa, Bid. aurea, Bid. beckii, Bid. Bipinnata, Bid. biternata, Bid. parviflora, Bid. connata und Bid. tripartita. Die Spezies Bid. alba, Bid. pilosa, Bid. bipinnata und Bid. parviflora sind bevorzugt werden erfindungsgemäß verwendet. Die Spezies Bid. alba und Bid. pilosa werden erfindungsgemäß verwendet.

Die Unterscheidung zwischen den genannten Spezies erfolgt bevorzugt an jungen und ausgewachsenen Pflanzen, die einen differenzierten Wuchs aufweisen und bereits Fruchtorgane, wie Knospen und Blüten, und in Farbe und Form differenzierte Blätter und Blüten aufweisen. Vorzugsweise werden ein- bis vierjährige, besonders bevorzugt ein bis dreijährige, Pflanzen verwendet.

Differenzierte Pflanzen der Spezies Bid. alba und Bid. pilosa unterscheiden sich in der Wuchshöhe, wobei Bid. alba eine geringere maximale Wuchshöhe von bis zu 2 m aufweist als Bid. pilosa. Die Blüten von Bid. alba sind klein und weisen eine radiale Symmetrie auf und haben ein zu den Gänseblümchen vergleichbares Erscheinungsbild mit einem gelben Blütenstaub im Blütenzentrum und fünf, insbesondere weißen, Blütenblättern. Die Blüten sind stets an der Spitze eines Zweiges oder Stängels angeordnet. Die weiteren Unterscheidungsmerkmale zu Bid. pilosa sind dem Fachmann bekannt.

Die vorgenannten Bidens-Spezies umfassen als sekundäre Pflanzenstoffe Flavonoide, Luteolin, Terpene, Polyacetylene, Phenylheptatriyn (PHT), Phenylpropanoide, insbesondere Anethol, Apiol, Zimtaldehyd, Dillapiol und Estragol, Lipide und Benzoide und weisen erfindungsgemäß eine antimikrobielle, vorzugsweise antibakterielle und/oder antimykotische, sowie eine anti-entzündliche Wirksamkeit auf.

In einer Ausführungsform der Erfindung umfasst die transiente Hautflora Staphylococcen, Streptococcen, Methicillin-resistente Staphylococcus aureus (MRSA), Pseudomonaden und/oder Acenitobakterien.

Im Sinne der Erfindung weisen die erfindungsgemäßen Bidens-Spezies, vorzugsweise Bid. alba und/oder Bid. pilosa, eine antimikrobielle, insbesondere mindestens antibakterielle, Wirksamkeit gegen eine transiente Hautflora auf, welche insbesondere Staphylococcen, Streptococcen, Methicillin-resistente Staphylococcus aureus (MRSA), Pseudomonaden und/oder Acenitobakterien umfasst. Besonders bevorzugt weisen die erfindungsgemäßen Bidens-Spezies, vorzugsweise Bid. alba und/oder Bid. pilosa, eine antibakterielle Wirksamkeit gegen Staphylococcus aureus (ATCC 25923), Staphylococcus epidermidis (ATCC 14990), MRSA (NCTC 10442), Pseudomonas aeruginosa (ATCC 27853) und Acenitobacter baumanii (ATCC BAA 747) auf (Beispiel 7).

Insbesondere wird eine antimikrobielle, vorzugsweise antibakterielle, Wirksamkeit durch Pflanzenextrakte einer Bidens-Spezies, vorzugsweise Bid. alba und/oder Bid. pilosa, erzielt, die gegen die Mikroorganismen, insbesondere gram-positive und/oder gram-negative Bakterien, aktive Verbindungen aufweisen, umfassend Centaurein, Centauredin, Polyacetylene, Phenylheptatriyn (PHT), Polyyne, 1,2-Dihydroxytrideca-3,5,7,9,11-Pentayn.

Im Sinne der Erfindung haben die vorgenannten Bidens-Spezies, vorzugsweise zusätzlich zur antimikrobiellen, insbesondere antibakteriellen, Wirksamkeit, eine anti-entzündliche Wirksamkeit bei kleiner gleich 200 ± 10 µg/ml des jeweiligen Pflanzenextraktes, vorzugsweise kleiner gleich 180 ± 10 µg/ml, kleiner gleich 160 ± 10 µg/ml, kleiner gleich 140 ± 10 µg/ml, besonders bevorzugt kleiner gleich 130 ± 10 µg/ml und kleiner gleich 110 ± 10 µg/ml, gemessen als $IC_{50}$ der 5-LOX Inhibierung.

Insbesondere wird eine anti-entzündliche Wirksamkeit durch Pflanzenextrakte einer Bidens-Spezies, vorzugsweise Bid. alba und/oder Bid. pilosa, erzielt, die gegen die 5-Lipoxygenase aktive Verbindungen enthalten, umfassend Triterpene, Flavonoide, Aurone, Chalkone, Luteolin, 1-Phenyl-1,3-diyn-5-en-7-ol-acetat, Kaffeate und Ethyl-Kaffeate.

Die Familie Verbenaceae, auch als Eisenkrautgewächse bekannt, umfasst etwa 35 Geni. Der b) Genus Stachytarpheta (=B) umfasst die Spezies Sta. angustifolia, Sta. cayennensis, Sta. chamissonis, Sta. glauca, Sta. glabra, Sta. jamaicensis, Sta. indica, Sta. mutabilis, Sta. steyermarkii, Sta. svensonii und Sta. urticaefolia. Spezies im Sinne der Erfindung sind Sta. cayennensis, Sta. jamaicensis und Sta. indica und besonders bevorzugt ist Sta. jamaicensis als ein Pflanzenextrakt im erfindungsgemäßen Phyto-Gemisch.

Die vorgenannten Stachytarpheta-Spezies enthalten als aktive Verbindungen die Inhaltsstoffe 3,4-Dihydroxyzimtsäure (Kaffeesäure), Flavonoide, Saponine, Tannine, Phenole, Steroide, insbesondere Scutellarein und Hispidulin, Terpene, Phenylpropanoide, insbesondere Verbascoside (auch als Acteoside bekannt), Glykoside, insbesondere Phenylethanoid-Glykoside, Phenylpropanoid-Glykoside, Iridoide, Iridoidglykoside, Ipolamiide, Tarphetalin, und 4-Methoxycarbonyl-7-methylcyclopenta[c]pyran (Fulvoipolamiide).

Verbascoside sind Phenylethanoid-Glykoside, die ein Ester aus dem Phenylethanoidhydroxytyrosol, der Phenylethanoid-3,4-dihydroxyzimtsäure und dem Zucker alpha-L-Rhamnopyranosyl-(1-3)-beta-D-Glycopyranose sind.

Als antimikrobiell, vorzugsweise antibakteriell und/oder antimykotisch, wirkende Verbindungen umfassen Stachytarpheta-Spezies die Verbindungen Verbascoside, Flavonoide, Glykoside, Phenylethanoid- und Phenylpropanoid-Glykoside und Anthrachinone.

Als anti-entzündlich wirkende Verbindungen umfassen Stachytarpheta-Spezies Verbascoside, Flavonoide, Iridoide, Ipolamiide, Iridoide-Ipolamiide, Acetoside, Fulvoipolamiide, Sesquiterpenlactone und Proazulene. Diese Verbindungen liegen insbesondere in den Blättern der erfindungsgemäßen Stachytarpheta-Spezies vor. Daher werden im Sinne der Erfindung bevorzugt Pflanzenbestandteile enthaltend die vorgenannten Verbindungen, insbesondere mindestens die Blätter, zur Herstellung eines Pflanzenextraktes, von Sta. jamaicensis, Sta. indica und/oder Sta. cayennensis, verwendet. Der vorgenannte Pflanzenextrakt wird vorzugsweise als ethanolischer Pflanzenextrakt mit größer gleich 70-% Ethanol gewonnen.

Für Bid. alba, Sta. jamaicensis und Bur. simaruba als jeweilige Vertreter für die erfindungsgemäßen Spezies des a), b), und c) wurden überraschenderweise jeweils eine signifikante anti-entzündliche Wirksamkeit nachgewiesen (Beispiel 6, Tabelle 2). Diese Wirksamkeit kann auf die in den erfindungsgemäßen Spezies enthaltenden und mittels des erfindungsgemäßen Verfahrens extrahierten Verbindungen zurückgeführt werden, umfassend Verbascoside, Flavonoide, Iridoide, Ipolamiide, Fulvoipolamiide Sesquiterpenlactone, Polyacetylene und/oder Proazulene.

Im Sinne der Erfindung weisen die Pflanzenextrakte der erfindungsgemäßen Stachytarpheta-Spezies eine gute antimikrobielle, mindestens eine antibakterielle, Wirksamkeit gegen eine transiente Hautflora auf, welche insbesondere Staphylococcen, Streptococcen, Methicillin-resistente Staphylococcus aureus (MRSA), Pseudomonaden und/oder Acenitobakterien umfasst. Besonders weisen Sta. cayennensis, Sta. jamaicensis und/oder Sta. indica eine antibakterielle Wirksamkeit gegen Staphylococcus aureus (ATCC 25923), Staphylococcus epidermidis (ATCC 14990), MRSA (NCTC 10442), Pseudomonas aeruginosa (ATCC 27853) und Acenitobacter baumanii (ATCC BAA 747) (Beispiel 7).

Im Sinne der Erfindung haben die erfindungsgemäßen Stachytarpheta-Spezies, vorzugsweise zusätzlich zur antimikrobiellen, vorzugsweise antibakteriellen und/oder antimykotischen, Wirksamkeit, eine anti-entzündliche Wirksamkeit bei kleiner gleich $200 \pm 10$ μg/ml des jeweiligen Pflanzenextraktes, vorzugsweise kleiner gleich $180 \pm 10$ μg/ml, kleiner gleich $160 \pm 10$ μg/ml, kleiner gleich $140 \pm 10$ μg/ml, kleiner gleich $120 \pm 10$ μg/ml, besonders bevorzugt kleiner gleich $100 \pm 10$ μg/ml, kleiner gleich $85 \pm 10$ μg/ml, kleiner gleich $80 \pm 10$ μg/ml, gemessen als $IC_{50}$ der 5-LOX Inhibierung.

Die Familie Burseraceae umfasst den Subtribus Burserinae, zu dem der c) Genus Bursera (=C) zugeordnet ist. Der Genus Bursera umfasst etwa 100 Spezies umfassend Bur. bipinnata, Bur. fagaroides, Bur. glabrifolia, Bur. malacophylla, Bur. microphylla, Bur. bolivarii, Bur. trifoliolata und Bur. simaruba. Im Sinne der Erfindung werden Bur. simaruba, Bur. microphylla und/oder Bur. glabrifolia verwendet. Besonders bevorzugt ist Bur. simaruba als ein Pflanzenextrakt im erfindungsgemäßen Phyto-Gemisch.

Im Sinne der Erfindung weisen die Pflanzenextrakte der erfindungsgemäßen Bursera-Spezies eine gute antimikrobielle, mindestens eine antibakterielle, Wirksamkeit gegen eine transiente Hautflora auf, welche insbesondere Staphylococcen, Streptococcen, Methicillin-resistente Staphylococcus aureus (MRSA), Pseudomonaden und/oder Acenitobakterien umfasst. Bur. simaruba, Bur. microphylla und/oder Bur. glabrifolia sind wirksam gegen Staphylococcus aureus (ATCC 25923), Staphylococcus epidermidis (ATCC 14990), MRSA (NCTC 10442), Pseudomonas aeruginosa (ATCC 27853) und Acenitobacter baumanii (ATCC BAA 747) auf (Beispiel 7).

Im Sinne der Erfindung haben die erfindungsgemäßen Bursera-Spezies, vorzugsweise zusätzlich zur antimikrobiellen Wirksamkeit, eine anti-entzündliche Wirksamkeit bei kleiner gleich $200 \pm 10$ μg/ml des jeweiligen Pflanzenextraktes, vorzugsweise kleiner gleich $180 \pm 10$ μg/ml, kleiner gleich $160 \pm 10$ μg/ml, kleiner gleich $140 \pm 10$ μg/ml, besonders bevorzugt kleiner gleich $135 \pm 10$ μg/ml, kleiner gleich $125 \pm 10$ μg/ml, gemessen als $IC_{50}$ der 5-LOX Inhibierung.

Eine antibakterielle Wirksamkeit wurde überraschend von Sta. jamaicensis und Bid. alba sowie vom Stem. maritima nachgewiesen (siehe Beispiel 7). Insbesondere weisen die vorgenannten Spezies eine signifikante Wirksamkeit gegen gram-positive Bakterien und besonders gegen MRSA auf. Somit weisen die hierin beschriebenen erfindungsgemäßen Phyto-Gemische mindestens eine antibakterielle und vorzugsweise zusätzlich eine anti-entzündliche Wirksamkeit auf.

In einer Ausführungsform weist das erfindungsgemäße Phyto-Gemisch eine anti-entzündliche Wirksamkeit bei kleiner gleich $200 \pm 10$ μg/ml auf, gemessen als IC50 der 5-LOX Inhibierung der jeweiligen Extraktmischung. Eine besonders bevorzugte Kombination im Sinne der Erfindung ist ein Phyto-Gemisch aus einem Pflanzenextrakt von a) Bid. alba und/oder Bid. pilosa und einem Pflanzenextrakt von b) Sta. jamaicensis, Sta. cayennensis und/oder Sta. indica. Die vorgenannten Kombinationen weisen überraschend eine erhöhte antibakterielle sowie anti-entzündliche Wirksamkeit auf (Beispiele 2 bis 8). Besonders überraschend wurde eine doppelte Wirksamkeit für alle vorgenannten bevorzugten Spezies festgestellt. Die hierin beschriebenen Versuche fassen exemplarisch die beschriebenen Wirksamkeiten für die jeweiligen erfindungsgemäßen Spezies des Genus a) Bidens, b) Stachytarpheta und c) Bursera zusammen.

Eine Ausführung des erfindungsgemäßen Phyto-Gemisches umfassend die jeweilige Kombination der ethanolischen Pflanzenextrakte, vorzugsweise trockener Extrakte, von

a) Bidens alba und/oder Bidens pilosa und
b) Stachytarpheta jamaicensis

weisen überraschend eine anti-entzündliche Wirksamkeit bei kleiner gleich 90 $\pm$ 10 $\mu$g/ml auf, vorzugsweise bei kleiner gleich 70 $\pm$ 10 $\mu$g/ml und besonders bevorzugt bei 50 $\pm$ 10 $\mu$g/ml, gemessen als $IC_{50}$ der 5-LOX Inhibierung (Tabelle 3). Das vorgenannte Phyto-Gemisch weist entgegen den Erwartungen gleichzeitig eine gute antimikrobielle, insbesondere antibakterielle, Wirksamkeit auf (Tabelle 5b). Entsprechende Wirksamkeiten weisen die anderen erfindungsgemäßen Spezies a), b) und c) auf, wie bereits oben aufgeführt.

Die Kombination aus b) Stachytarpheta jamaicensis und c) Bursera simaruba wird beschrieben.

Eine Ausführung des erfindungsgemäßen Phyto-Gemisches umfassend die Kombination der ethanolischen Pflanzenextrakte, vorzugsweise nachfolgend getrockneter Extrakte, von

a) Bidens alba und c) Bursera simaruba

weisen eine anti-entzündliche Wirksamkeit bei kleiner gleich 90 $\pm$ 10 $\mu$g/ml auf, vorzugsweise bei kleiner gleich 80 $\pm$ 10 $\mu$g/ml, gemessen als $IC_{50}$ der 5-LOX Inhibierung (Tabelle 3). Das vorgenannte Phyto-Gemisch weist gleichzeitig eine gute antimikrobielle, insbesondere antibakterielle, Wirksamkeit auf. Entsprechende Wirksamkeiten weisen die anderen jeweils erfindungsgemäßen Spezies des Genus a) Bidens und c) Bursera auf.

Besonders bevorzugte Kombinationen aus a) und b) und/oder c) und optional d) umfassen

i) mindestens einen ethanolischen Pflanzenextrakt mit einer anti-entzündlichen Wirksamkeit und mindestens einen zweiten ethanolischen Pflanzenextrakt mit einer antimikrobiellen Wirksamkeit,
ii) mindestens einen ethanolischen Pflanzenextrakt mit einer anti-entzündlichen Wirksamkeit und gleichzeitig mit einer antimikrobiellen Wirksamkeit,
iii) mindestens einen ethanolischen Pflanzenextrakt mit einer anti-entzündlichen Wirksamkeit und gleichzeitig mit einer antimikrobiellen Wirksamkeit und mindestens einen weiteren Extrakt einer biologisch aktiven Pflanze d),

wobei jeweils bevorzugt Ethanolextrakte, größer gleich 70 % Ethanol, eingesetzt werden und insbesondere nach Trocknung mit einem Rest-Ethanolgehalt kleiner gleich 5 %, kleiner gleich 1 %, besonders bevorzugt kleiner gleich 0,1 %. Die oben beschriebenen Ausführungen zu den Inhaltsstoffen der einzelnen Pflanzenspezies gelten hier entsprechend.

Bevorzugte Formulierungen des vorbeschriebenen Phyto-Gemisches und dessen Kombinationen umfassen Tee, Tinktur, Lösung, Dispersion und Suspension, Pulver und halbfeste Formen, wie Salben, Cremes und Pasten. Der erfindungsgemäße Tee basiert auf mindestens einem trockenen und ethanolfreien Pflanzenextrakt aus a) und mindestens einem weiteren ethanolischen Pflanzenextrakt ausgewählt aus b) und/oder c) und optional d).

Als weitere d) biologisch aktive Pflanzen im Sinne der Erfindung werden Spezies des Genus Stemodia (=D1) eingesetzt. Diese wurden früher der Familie Scrophariceae zugeordnet und heute der Familie Plantaginaceae, die der Ordnung Lamiales zugehörig ist. Dem Genus Stemodia (Stem.) gehören etwa 40 Spezies an umfassend Stem. maritima, Stem. lantana und Stem. durantifolia, die im Sinne der Erfindung eingesetzt werden. Besonders bevorzugt ist Stem. maritima.

Als weitere d) biologisch aktive Pflanzen werden auch Spezies des Genus Aloe (=D2) eingesetzt, die der Unterfamilie Asphodellideae zugeordnet ist, die der Familie Xanthorrhoeaceae angehört. Der Genus Aloe umfasst etwa 500 Spezies. Erfindungsgemäß werden vorzugsweise Aloe vera, Aloe barbadensis, Aloe albifora, Aloe perfoliata, Aloe vulgaris, Aloe indica und/oder Aloe chinensis eingesetzt. Besonders bevorzugt ist Aloe vera mit oder ohne Anthrachinone.

Eine Mischung aus einem ethanolischen Pflanzenextrakt von a) Bid. alba, Bid. pilosa, und mindestens einem weiteren ethanolischen Pflanzenextrakt ausgewählt aus b) Sta. jamaicensis, Sta. cayennensis, Sta. indica und/oder c) Bur. simaruba, Bur. microphylla, Bur. glabrifolia und optional d) eine Aloe-Spezies (D2), bevorzugt Aloe vera weist besonders gute Wirksamkeiten bei topischer Anwendung auf der Haut, Schleimhaut und Mundschleimhaut auf, insbesondere zur Prävention und Behandlung von Effloreszenzen im Sinne der Erfindung. Tabelle 1a fasst geeignete Kombinationen der bevorzugten Spezies zusammen.

Tabelle 1a

| Extrakt-Kombination | Bevorzugte Spezies | Optionale bevorzugte Spezies von | Optionale bevorzugte Spezies von |
|---|---|---|---|
| A-E / B-E | Bi. alba/ Sta. jamaicensis | C-E | D1 und/oder D2 |
| A-E / C-E | Bid. alba/Bur. simaruba | B-E | D2 = Aloe vera |
| B-E / C-E | Sta. jamaicensis/ Bur. simaruba | A-E | D1 = Stem. maritima und ggf. D2 |
| A-E / D1-E | Bid. alba/ Stem. maritima | B-E | D2 |
| B-E / D1-E | Sta. jamaicensis/ Stem. maritima | A-E | D2 |
| C-E/D1-E | Bur. simaruba/ Stem. maritima | A-E | D2 |
| A-E / D2 | Bid. alba / Aloe vera | B-E | D1 |
| B-E / D2 | Sta. jamaicensis / Aloe vera | C-E | D1 |
| C-E / D2 | Bur. simaruba / Aloe vera | A-E | D1 |

Wobei A = Genus Bidens, B=Genus Stachytarpheta, C=Genus Bursera, D1= Genus Stemodia, D2= Genus Aloe und E= ethanolischer Extrakt.

Der Anteil des mindestens einen Pflanzenextraktes aus a) und b) und/oder c) und optional d) beträgt, bezogen auf das jeweilige Trockengewicht (ad 100 Gew.-%), größer gleich 1 Gew.-% vorzugsweise bis kleiner gleich 50 Gew.-%, bevorzugt größer gleich 3 Gew.-%, größer gleich 5 Gew.-%, größer gleich 7 Gew.-% bis jeweils kleiner gleich 50 Gew.-%, wobei der Gesamtgehalt des mindestens einen Pflanzenextraktes oder der Kombination aus a) und b) und/oder c) bevorzugt größer gleich 3 Gew.-% beträgt, bezogen auf den Gesamtgehalt des Phyto-Gemisches (ad 100 Gew.-%), vorzugsweise größer gleich 5 Gew.-%, größer gleich 7 Gew.-% bis kleiner gleich 50 Gew.-%, vorzugsweise kleiner gleich 30 Gew.-% beträgt. Bei Zugabe eines d) Aloe-Extraktes (D2) beträgt der Anteil an D2 vorzugsweise größer gleich 5-Gew.-%, größer gleich 10 Gew.-%, größer gleich 15 Gew.-%, größer gleich 20 Gew.-%, bis jeweils kleiner gleich 70 Gew.-%. Die Anteile der anderen Pflanzenextrakte a) und b) und/oder c) sind entsprechend geringer (Beispiel 8b). Die hier beschriebenen bevorzugten Anteile gelten entsprechend für die nachfolgend beschriebenen Zusammensetzungen und Formulierungen.

Bei Kombination von zwei Pflanzenextrakten werden bevorzugt Verhältnisse von 1:1 Mischungen angefertigt, wie auch in den nachstehenden Beispielen 2 bis 8 untersucht. Die Verhältnisse können variabel eingestellt werden zwischen 1:10 bis 10:1 bei Kombination von zwei Pflanzenextrakten. Bei drei oder mehr Pflanzenextrakten werden bevorzugt Verhältnisse von 1:1:1 usw. eingesetzt. Die Mengen-Verhältnisse können variabel eingestellt werden, abhängig von der Verfügbarkeit des Materials und der Verträglichkeit des Patienten für den jeweiligen Extrakt. So kann bei Vorliegen einer Allergie gegen eine der hierin beschriebenen Pflanzen oder Unverträglichkeit gegenüber einem der vorbeschriebenen Inhaltsstoffe, diese aus dem Phyto-Gemisch ausgenommen werden. Zur Erzielung der gewünschten Wirksamkeit kann dafür der Gehalt der anderen Pflanzenextrakte entsprechend angepasst werden.

In einer Ausführungsform weist das erfindungsgemäße Phyto-Gemisch vorzugsweise eine antibakterielle und/oder antimykotische Wirksamkeit gegen eine transiente Hautflora und eine anti-entzündliche Wirksamkeit auf.

"Antimikrobielle Wirksamkeit" im Sinne der Erfindung umfasst die Hemmung und Reduzierung der Vermehrungsfähigkeit, Teilungsfähigkeit und Reproduktion der Mikroorganismen bis hin zur Inaktivierung oder Abtöten der Mikroorganismen. "Antimikrobiell" umfasst mindestens antibakteriell (gegen gram-positive und -negative Bakterien) und vorzugsweise zusätzlich antimykotisch (gegen Pilze und Hefen). Eine antimikrobielle Wirksamkeit umfasst weiterhin die Hemmung des Biosynthese-Apparates der Mikroorganismen, wie z.B. der Synthese von Toxinen, Pathogenitätsfaktoren und anderer physiologischer Reaktionen oder eine Immunantwort auslösender Verbindungen. Toxine umfassen Exotoxine und Endotoxine. Insbesondere Exotoxine, wie die Pilzgifte Aflatoxine oder Exofilantin A und B von Staphylococcus aureus.

Vorzugsweise umfasst "antimikrobielle Wirksamkeit" mindestens eine Antibiotika-analoge Wirksamkeit, insbesondere im geringeren Ausmaß und vorzugsweise mit weniger oder keinen Nebenwirkungen im Vergleich zu synthetischen Antibiotika.

Eine "transiente Hautflora" im Sinne der Erfindung bedeutet, eine mikrobiologische Flora auf/in der Haut und/oder Mundschleimhaut, die durch Einwirkung diverser Einflussfaktoren zu einer Effloreszenz führen kann, eine Effloreszenz resultiert oder durch systemische Streuung weitere Gewebe schädigen kann.

Die Hautflora einer gesunden Haut umfasst eine "residente Hautflora", die auch als physiologische, körpereigene Hautflora oder auch "Standortflora" bezeichnet wird. Die "residente Hautflora" umfasst Keime, die dem gesunden Organismus nicht schaden, umfassend Staphylococcen, insbesondere Staphylococcus epidermidis, Propioni-, Myko- und Corynebakterien. Diese Keime vermehren sich vorzugsweise im Stratum corneum und bilden eine Schutzflora gegen pathogene und fremde Mikroorganismen. Die Zusammensetzung der residenten Hautflora kann für unterschiedliche Hautregionen, wie Gesicht, Hände, Füße, Kopf, Außenohr sowie Lippen und Mundschleimhaut, die Haut des Auges, insbesondere die Hornhaut, Bindehaut und Schleimhaut des Auges, variabel sein.

Davon abzugrenzen ist die transiente, somit vorübergehende, Hautflora umfassend pathogene Keime. Pathogene Keime umfassen gram-negative Coccen, wie Neisseria, Gonococcus, Meningococcus, gram-positive Coccen, wie Staphylococcen, Streptococcen, insbesondere Streptococcus aureus, Mikrococcen, Enterobakterien, Pneumococcen und Clostridien, gram-negative Stäbchen, wie Bordetella, Campylobacter, Hämophilus, Heliobacter, Legionella, Salmonella, Shigella, Vibrio, Yersinia, Escherichia coli, Klebsiella, Proteus und Pseudomonaden, gram-positive Stäbchen, wie Bacillus, Clostridium, Corynebacterium, Listeria und Mykobakterien und/oder Hefen, Pilze, Candida und/oder Viren.

Eine gesunde Hautflora kann durch unterschiedliche Einflussfaktoren, wie physikalische, chemische, pathogene und körpereigene physiologische Einflussfaktoren beeinträchtigt werden und in eine transiente Hautflora übergehen. Insbesondere können Fremdkeime oder pathogene Keime die gesunde Hautflora stören oder verdrängen und sich somit vermehren und ausbreiten, was letztendlich zu Hauterkrankungen führen kann. Insbesondere Erkrankungen der Cutis, vorzugsweise der Epidermis umfassend mindestens eine Veränderung mindestens eines Gewebes ausgewählt aus Stratum corneum, Stratum lucidum, Stratum granulosum, Stratum spinosum, Stratum basale und/oder Stratum germinativum (= Stratum spinosum + Stratum basale), Dermis umfassend Stratum paillare und Stratum reticulare und/oder der Subcutis umfassend Bindegewebe mit Fibroblasten, Endothelzellen, Kollagen und Fettzellen.

Folglich umfasst eine transiente Hautflora erfindungsgemäß eine gestörte Hautflora umfassend mindestens einen der vorgenannten Keime, die phänotypisch zu Effloreszenzen mindestens eines der vorgenannten Gewebe auf der Haut und Mundschleimhaut führen kann.

Physikalische Einflussfaktoren umfassen die physikalische Störung und Schädigung der Hautstruktur und umfassen Verletzungen, Stiche, Schnitte, Kratzer, Schürfungen, Verbrennungen oder Verätzungen mindestens der Epidermis umfassend Stratum corneum, Stratum lucidum, Stratum granulosum, Stratum spinosum, Stratum basale und/oder Stratum germinativum (= Stratum spinosum + Stratum basale), optional der Dermis umfassend Stratum paillare und Stratum reticulare und/oder der Subcutis umfassend Bindegewebe mit Fibroblasten, Endothelzellen, Kollagen und Fettzellen.

In einer besonderen Ausführung der vorliegenden Erfindung weist das erfindungsgemäße immunologisch aktive Phyto-Gemisch umfassend mindestens einen erfindungsgemäßen ethanolischen Pflanzenextrakt ausgewählt aus a) und mindestens einen weiteren ethanolischen Pflanzenextrakt ausgewählt aus b) und/oder c) und optional d) mit einer antimikrobiellen, vorzugsweise antibakteriellen, Wirksamkeit gegen eine transiente Hautflora auf, wobei die transiente Hautflora Staphylococcen, Streptococcen, Methicillin-resistente Staphylococcus aureus (MRSA), Pseudomonaden und/oder Acenitobakterien umfasst.

In einer besonderen Ausführung der vorliegenden Erfindung umfasst das erfindungsgemäße immunologisch aktive Phyto-Gemisch mindestens einen Pflanzenextrakt, der eine antibakterielle Wirksamkeit gegen mindestens einen der vorgenannten Keime einer transienten Hautflora bei einer Konzentration größer gleich 10 μg/ml bis kleiner gleich 10 mg/ml aufweist, gemessen als minimale Hemmkonzentration (MIC) und/oder als minimale bakterizide Konzentration (MBC) des jeweiligen Pflanzenextrakts oder der Extraktmischung. Vorzugsweise weist der mindestens eine Pflanzenextrakt eine antibakterielle Wirksamkeit bei einer Konzentration größer gleich 10 μg/ml bis kleiner gleich 10 mg/ml, größer gleich 10 μg/ml bis kleiner gleich 8 mg/ml, größer gleich 100 μg/ml bis kleiner gleich 8 mg/ml, größer gleich 250 μg/ml bis kleiner gleich 8 mg/ml, vorzugsweise größer gleich 500 μg/ml bis kleiner gleich 8 mg/ml, insbesondere größer gleich 10 μg/ml bis kleiner gleich 6 mg/ml, größer gleich 100 μg/ml bis kleiner gleich 6 mg/ml, größer gleich 250 μg/ml bis kleiner gleich 6 mg/ml, größer gleich 500 μg/ml bis kleiner gleich 6 mg/ml, größer gleich 10 μg/ml bis kleiner gleich 4 mg/ml, größer gleich 100 μg/ml bis kleiner gleich 4 mg/ml, größer gleich 250 μg/ml bis kleiner gleich 4 mg/ml, größer gleich 500 μg/ml bis kleiner gleich 4 mg/ml.

In einer Ausführungsform umfasst das erfindungsgemäße Phyto-Gemisch eine antibakterielle Wirksamkeit bei einer Konzentration von größer gleich 10 μg/ml bis kleiner gleich 10 mg/ml, gemessen als minimale Hemmkonzentration (MHK/MIC) und/oder als minimale bakterizide Konzentration (MBK/MBC) der jeweiligen Extraktmischung. In einer besonderen Ausführungsform weist das erfindungsgemäße Phyto-Gemisch mit den nachfolgenden bevorzugten Kombinationen von Pflanzenextrakten eine antimikrobielle, vorzugsweise antibakterielle, Wirksamkeit auf, bei einer Konzentration größer gleich 100 μg/ml bis kleiner gleich 10 mg/ml (MIC und/oder MBC) umfassend

i) Sta. jamaicensis, cayennensis und/oder indica und Bid. alba und/oder pilosa

ii) Sta. jamaicensis, cayennensis und/oder indica und Bur. simaruba und Bidens alba/pilosa

iii) Sta. jamaicensis und/oder indica und Bur. simaruba, Bur. microphylla und Bur. glabrifolia und Bid. alba und/oder pilosa und

iv) i), ii) oder iii) und jeweils eine Aloe-Spezies und optional Stemodia maritima

Die vorgenannten Phyto-Gemische weisen überraschend gute Wirksamkeiten gegen MRSA auf, wie in Beispiel 7 gezeigt (Tabelle 5a und 5b). Überraschend wurde exemplarisch für ausgewählte Spezies a), b) und c) der erfindungsgemäßen Pflanzenextrakte eine antibakterielle Wirksamkeit nachgewiesen. Entsprechendes gilt für die anderen erfindungsgemäßen Spezies des Genus a), Bidens, b) Stachytarpheta und c) Bursera.

Das erfindungsgemäße immunologisch aktive Phyto-Gemisch umfassend mindestens einen der vorbeschriebenen erfindungsgemäßen Pflanzenextrakte ausgewählt aus a) und mindestens einen weiteren ethanolischen Pflanzenextrakt ausgewählt aus b) und/oder c) weist eine anti-entzündliche Wirksamkeit bei kleiner gleich $200 \pm 10$ $\mu$g/ml auf, vorzugsweise bei kleiner gleich $180 \pm 10$ $\mu$g/ml, kleiner gleich $160 \pm 10$ $\mu$g/ml, kleiner gleich $140 \pm 10$ $\mu$g/ml, kleiner gleich $130 \pm 10$ $\mu$g/ml besonders bevorzugt bei kleiner gleich $120 \pm 10$ $\mu$g/ml, gemessen als $IC_{50}$ der 5-LOX Inhibierung, des jeweiligen Pflanzenextrakts oder der Extraktmischung. Besonders bevorzugte Ausführungen weisen eine anti-entzündliche Wirksamkeit bei kleiner gleich $100 \pm 10$ $\mu$g/ml auf, vorzugsweise bei kleiner gleich $90 \pm 10$ $\mu$g/ml, kleiner gleich $80 \pm 10$ $\mu$g/ml, kleiner gleich $70 \pm 10$ $\mu$g/ml und kleiner gleich $60 \pm 10$ $\mu$g/ml oder kleiner gleich $50 \pm 10$ $\mu$g/ml.

Besonders bevorzugt weist der mindestens eine Pflanzenextrakt eine anti-entzündliche Wirksamkeit bei kleiner gleich $90 \pm 10$ $\mu$g/ml des jeweiligen Pflanzenextrakts der erfindungsgemäßen Spezies a) und b) und/oder c) auf.

Bevorzugt umfasst das immunologisch aktive Phyto-Gemisch, mindestens einen Pflanzenextrakt ausgewählt aus a) Bid. alba, Bid. pilosa und b) Sta. jamaicensis, Sta. indica, Sta. cayennensis und/oder c) Bur. microphylla, Bur. glabrifolia und/oder Bur. simaruba und optional d) als Auszug der Inhaltsstoffe der jeweiligen Pflanze, vorzugsweise der oberirdischen Bestandteile der mindestens einen Pflanze. Vorzugsweise umfasst der Auszug Inhaltsstoffe aus unverdorbenen, unveränderten, frisch geernteten oder getrockneten Pflanzenteilen umfassend Rinde, Stängel, Zweige, Äste, Blätter, Blüte, Knospen, Samen, Hüllen, Pollen, Fruchtorgane, photosynthetisch aktive Bestandteile und/oder Speicherorgane. Der Auszug aus getrockneten Pflanzenbestandteilen ist bevorzugt. Der Vorteil eines Auszugs aus getrockneten Pflanzenbestandteilen ist die Abwesenheit des Wassers, sodass höher konzentrierte Auszüge der jeweiligen Pflanze erhalten werden. Damit werden Extrakte mit vergleichbar höheren Gehalten an den aktiven Verbindungen pro Maßeinheit des jeweiligen Auszugs erhalten.

Folglich weisen die aus getrockneten Pflanzen erfindungsgemäß erhaltenen Pflanzenextrakte einen erhöhten Anteil an den vorbeschriebenen Verbindungen auf, vorzugsweise mindestens eine antibakteriell und/oder anti-entzündlich aktive Verbindungen umfassend Flavonoide, Terpene, Benzoide, Phenylpropanoide, Glykoside und Verbascoside, Iridoide, Ipolamiide, Fulvopolamiide, Sesquiterpenlactone und/oder Proazulene.

"Inhaltsstoffe" im Sinne der Erfindung umfassen alle der hierin beschriebenen pflanzlichen biologisch und/oder physiologisch aktiven Verbindungen.

In einer weiteren Ausführung der vorliegenden Erfindung umfasst das immunologisch aktive Phyto-Gemisch mindestens einen ethanolischen Pflanzenextrakt ausgewählt aus a) und mindestens einen weiteren ethanolischen Pflanzenextrakt ausgewählt aus b) und/oder c) und optional d), der jeweils einen Auszug mindestens einer der nachfolgenden Verbindungen, insbesondere der Inhaltstoffe, umfasst: Flavonoide, Saponine, Iridoide, Phenolsäuren, Polyphenole, Polysaccharide, Glycosilate, Terpene, Monoterpene, Sesquiterpenlactone, Proazulene, Sulfide, Carotinoide, Vitamine A B C D E, Aminosäuren, und/oder Mineralien. Besonders bevorzugt weisen ethanolische, getrocknete Pflanzenextrakte von a) Bid. alba, Bid. pilosa, b) Sta. jamaicensis, Sta. cayennensis, Sta. indica, c) Bur. microphylla, Bur. glabrifolia, Bur. simaruba und/oder d) Stem. maritima eine der vorgenannten Verbindungen auf.

In einer besonderen Ausführung des erfindungsgemäßen immunologisch aktiven Phyto-Gemisches umfasst dieses mindestens einen ethanolischen Pflanzenextrakt ausgewählt aus a) und mindestens einen weiteren ethanolischen Pflanzenextrakt ausgewählt aus b) und/oder c) und optional d), der mindestens eine der antimikrobiell, vorzugsweise antibakteriell, wirkenden Verbindungen Verbascoside, Flavonoide, Glykoside, Phenylethanoid-Glykoside, Phenylpropanoid-Glykoside und/oder Anthrachinone umfasst. Vorzugsweise umfasst das vorgenannte Phyto-Gemisch mindestens einen Pflanzenextrakt aus b) St. jamaicensis, Sta. cayennensis, Sta. indica, a) Bid. alba und/oder Bid. pilosa.

In einer weiteren Ausführung des erfindungsgemäßen immunologisch aktiven Phyto-Gemisches umfasst dieses mindestens einen ethanolischen Pflanzenextrakt ausgewählt aus a) und mindestens einen weiteren ethanolischen Pflanzenextrakt ausgewählt aus b) und/oder c) und optional d), der mindestens eine der anti-entzündlich wirkenden Verbindungen Verbascoside, Flavonoide, Iridoide, Ipolamiide, Fulvoipolamiide, Sesquiterpenlactone und/oder Proazulene umfasst. Vorzugsweise umfasst das vorgenannte Phyto-Gemisch mindestens einen Pflanzenextrakt aus b) Sta. jamaicensis, Sta. cayennensis, Sta. indica, a) Bid. alba und/oder Bid. pilosa.

In einer besonderen Ausführung des erfindungsgemäßen immunologisch aktiven Phyto-Gemisches umfasst dieses mindestens zwei der vorgenannten Pflanzenextrakte ausgewählt aus a) und mindestens einen weiteren ethanolischen Pflanzenextrakt ausgewählt aus b) und/oder c) und optional d) vorzugsweise umfassend mindestens eine der Spezies b) Sta. jamaicensis, Sta. cayennensis, und/oder Sta. indica, und eine der Spezies b) Bid. alba und/oder Bid. pilosa, welche ethanolische Auszüge der vorgenannten Inhaltsstoffe enthalten.

Die vorbeschriebenen, erfindungsgemäßen Pflanzenextrakte, Phyto-Gemische enthaltend die Pflanzenextrakte und erfindungsgemäßen Ausführungen werden zur Herstellung von Arzneimitteln, Medizinprodukten, Nahrungsergänzungsmitteln und Kosmetika bereitgestellt. Für diese Zwecke liegt das Phyto-Gemisch in unterschiedlichen Formen vor.

Daher ist ein weiterer Gegenstand der vorliegenden Erfindung das erfindungsgemäße immunologisch aktive Phyto-Gemisch, wobei der mindestens eine Pflanzenextrakt a) Bid. alba, Bid. pilosa, und b) Sta. jamaicensis, Sta. indica, Sta. cayennensis, und/oder c) Bur. microphylla, Bur. glabrifolia und/oder Bur. simaruba und optional d) vorliegt in

- flüssiger Form umfassend Lösung, Dispersion, Suspension, Emulsion, Tinktur, Sirup, Saft und Tee
- fester Form umfassend Tablette, Pulver, Puder, Dragee, Globuli, Granulat und Lyophylisat, Kapsel, insbesondere gefriergetrocknete Form, oder
- als Gemisch umfassend Kapseln, Aerosol, Spray, Emulsion, Lotion und Creme.

In einer Ausführungsform des immunologisch aktiven Phyto-Gemisches weist das erfindungsgemäße Phyto-Gemisch in flüssiger Form, vorzugsweise Tinktur, Lösung, Dispersion und/oder Suspension, jeweils alternativ einen für die Haut verträglichen pH größer gleich 3 bis kleiner gleich 9 auf, einen für die Mundschleimhaut verträglichen pH größer gleich 6 bis kleiner gleich 8, einen für die Nasenschleimhaut verträglichen pH größer gleich 5 bis kleiner gleich 7 oder einen für das Auge verträglichen pH größer gleich 7 bis kleiner gleich 9.

Nach Herstellung des erfindungsgemäßen Pflanzenextrakts, insbesondere Phyto-Gemisches, gemäß des erfindungsgemäßen Verfahrens liegt das unmittelbare Erzeugnis nach der Gefriertrocknung in gefriergetrockneter Form, vorzugsweise als Lyophilisat, vor. Anschließend kann es zu feinen Pulvern, Tabletten oder flüssigen Formen verarbeitet werden.

In einer Ausführung liegt das flüssige erfindungsgemäße immunologisch aktive Phyto-Gemisch als Tinktur vor, insbesondere als Lösung, Suspension oder Dispersion, umfassend

- größer gleich 1 Gew.-%, vorzugsweise größer gleich 3 Gew.-%, größer gleich 5 Gew.-%, größer gleich 7 Gew.-%, bezogen auf den Gesamtgehalt der Tinktur (T = 100 Gew.-%), mindestens eines ethanolischen Pflanzenextraktes ausgewählt aus a) Bid. alba, Bid. pilosa, und einen weiteren ethanolischen Pflanzenextrakt ausgewählt aus b) Sta. jamaicensis, Sta. cayennensis, Sta. indica und/oder c) Bur. simaruba, Bur. microphylla, Bur. glabrifolia,
- mindestens ein Säuerungsmittel umfassend Essigsäure, Citronensäure, Ascorbinsäure, Adipinsäure, Weinsäure, Mandelsäure, und/oder Apfelsäure,
- größer gleich 1 Gew.-%, vorzugsweise größer gleich 3 Gew.-%, größer gleich 5 Gew.-%, größer gleich 10 Gew.-%, größer gleich 15 Gew.-%, eines Aloe-Extrakts, bezogen auf den Gesamtgehalt der Tinktur (T = 100 Gew.-%),

wobei die Tinktur einen pH-Wert größer gleich 3 bis kleiner gleich 9 aufweist und die Tinktur ein wässrig/ethanolisches Gemisch ist mit einer Ethanolkonzentration größer gleich 70 %, bezogen auf die Gesamtzusammensetzung der Tinktur (z.B. Beispiel 8b). Bevorzugt beträgt der Gesamtgehalt des Pflanzenextrakts oder der Kombination aus jeweils einem Pflanzenextrakt a) und mindestens einen weiteren ethanolischen Pflanzenextrakt ausgewählt aus b) und/oder c) größer gleich 3 Gew.-%, vorzugsweise größer gleich 5 Gew.-%, größer gleich 7 Gew.-%, jeweils bezogen auf das Phyto-Gemisch (ad 100 Gew.-%).

Als Säuerungsmittel sind alle physiologisch verträglichen, synthetischen, biobasierten und natürlichen Säuerungsmittel geeignet. Insbesondere als Lebensmittel verfügbare Säuerungsmittel, wie Essig, Apfelessig, Weißweinessig, Balsamico-essig können als Säuerungsmittel eingesetzt werden. Bevorzugt werden hochreine, pharmazeutische und/oder kosmetisch zugelassene Säuerungsmittel eingesetzt.

Der geeignete pH-Wert des jeweiligen Phyto-Gemisches ist von der Art der Indikation und seiner Verabreichungsform abhängig. Zur Einstellung des pH-Wertes ist das Säuerungsmittel geeignet, ohne es auf diese Funktionen zu beschränken. Der pH-Wert beträgt größer gleich 3 bis kleiner gleich 9, vorzugsweise größer gleich 3,1, größer gleich 3,2, größer gleich 3,5 bis jeweils kleiner gleich 8,7, kleiner gleich 8,5 8,3 8,1 7,9, kleiner gleich 7,8 und vorzugsweise kleiner gleich 7,6.

Topische Formulierungen, vorzugsweise Lösung, Dispersion, Suspension, Tinktur, Aerosol, Spray und/oder halbfeste Formulierungen, wie Salbe, Creme und Paste, zur Anwendung auf der Haut im Sinne der Erfindung, ausgenommen Mund-/Nasenschleimhaut, Auge, Hornhaut, Bindehaut und Schleimhaut des Auges, enthaltend das erfindungsgemäße Phyto-Gemisch umfassend mindestens einen ethanolischen Pflanzenextrakt a) und mindestens einen weiteren ethanolischen Pflanzenextrakt ausgewählt aus b) und/oder c) und optional d), weisen jeweils bevorzugt einen pH größer gleich 3 bis kleiner gleich 9 auf, vorzugsweise größer gleich 3,5 bis kleiner gleich 8,7, kleiner gleich 8,5 8,3 8,1 7,9.

Insbesondere jeweils pH 3,2 3,4 3,6 3,8 4,0 4,2 4,4 4,6 4,8 5,0 5,2 5,3 5,4 5,5 5,6 5,7 5,8 5,9 6,0 6,2 6,4 6,6 6,8 7,0 7,2 7,4 7,6 7,8 8,0 8,1 8,3 8,5 oder 8,7.

Erfindungsgemäße Formulierungen (Beispiel 8a-b) zur Anwendung am Außenohr und Gehörgang, bevorzugt Lösung, Dispersion, Suspension, Tinktur, Spray, Aerosol und halbfeste Formulierungen, weisen jeweils einen pH größer gleich 3 bis kleiner gleich 9 auf, vorzugsweise größer gleich 4 bis kleiner gleich 7.

Erfindungsgemäße Formulierungen (Beispiel 8a-b) zur Anwendungen auf der Mundschleimhaut, bevorzugt Lösung, Dispersion, Suspension, Tinktur, Spray, Aerosol und halbfeste Formulierungen, weisen jeweils einen pH von bevorzugt größer gleich 6 bis kleiner gleich 8 auf, vorzugsweise größer gleich 6,2 bis kleiner gleich 7,8. Insbesondere 6,2 6,3 6,4 6,5 6,6 6,5 6,6 6,7 6,8 6,9 7,0 7,1 7,2 7,3 7,4 7,5 oder 7,6.

Erfindungsgemäße Formulierungen zum Aufbringen auf die Nasenschleimhaut, vorzugsweise Tinktur, Lösung, Dispersion, Suspension, Spray, Aerosol und halbfeste Formulierungen, weisen jeweils einen pH von bevorzugt größer gleich 5 bis kleiner gleich 7 auf. Insbesondere pH 5,1 5,2 5,3 5,4 5,5 5,5 5,6 5,7 5,8 5,9 6,0 6,1 6,2 6,3 6,4 6,5 6,6 6,7 6,8 6,9 oder 7,0.

Erfindungsgemäße Formulierungen, vorzugsweise Lösung, Dispersion, Suspension und Tinktur, zum Auf-/Einbringen auf/in das Auge, insbesondere auf die Hornhaut, Bindehaut und Schleimhaut des Auges, weisen jeweils einen physiologischen pH-Wert auf, bevorzugt größer gleich 7 bis kleiner gleich 9. Insbesondere pH 7,1 7,2 7,3 7,4 7,5 7,5 7,6 7,7 7,8 7,9 8,0 8,1 8,2 8,3 8,4 oder 8,5.

Die vorbeschriebenen pH-Werte und bevorzugten pH-Bereiche für die jeweiligen Anwendungen gelten entsprechend für alle beschriebenen Ausführungsformen des erfindungsgemäßen Phyto-Gemisches und dessen Formulierungen umfassend mindestens einen ethanolischen Pflanzenextrakt a) und mindestens einen weiteren ethanolischen Pflanzenextrakt ausgewählt aus b) und/oder c) und optional d) ohne explizit darauf einzugehen.

Eine weitere Ausführung des erfindungsgemäßen immunologisch aktiven Phyto-Gemisches umfasst in fester Form mindestens einen im Wesentlichen trockenen, insbesondere lösungsmittelfreien, Pflanzenextrakt ausgewählt aus a) Bid. alba, Bid. pilosa, b) Sta. indica, Sta. jamaicensis, Sta. cayennensis, und/oder c) Bur. simaruba, Bur. microphylla, Bur. glabrifolia, und optional d) Stemodia maritima, und/oder eine Aloe-Spezies.

"Im Wesentlichen trocken" im Sinne der Erfindung bedeutet, dass der jeweilige Pflanzenextrakt a) und b) und/oder c) und optional d) der mindestens einen Pflanze eine Restfeuchte hat von kleiner gleich 10 Gew.-%, vorzugsweise kleiner gleich 8 Gew.-%, kleiner gleich 5 Gew.-%, kleiner gleich 4 Gew.-%, kleiner gleich 3 Gew.-%, kleiner gleich 2 Gew.-%, besonders bevorzugt kleiner gleich 1 Gew.-%, kleiner gleich 0,1 Gew.-%, gleich 0 Gew.-%, bezogen auf das Gesamtgewicht des jeweiligen Pflanzenextraktes.

"Lösungsmittelfrei" im Sinne der Erfindung bedeutet einen Restlösungsmittelgehalt kleiner gleich 10 Gew.-%, vorzugsweise kleiner gleich 8 Gew.-%, kleiner gleich 5 Gew.-%, kleiner gleich 4 Gew.-%, kleiner gleich 3 Gew.-%, kleiner gleich 2 Gew.-%, kleiner gleich 1 Gew.-%, besonders bevorzugt kleiner gleich 0,1 Gew.-%, bezogen auf das Gesamtgewicht des jeweiligen Pflanzenextraktes der mindestens einen Pflanze, wobei Lösungsmittel wässrigen Ethanol, Ethanol, insbesondere größer gleich 70%-ig bis kleiner gleich 100 %-igen Ethanol, größer gleich 70%-igen Ethanol umfassen. Bevorzugt werden Lösungsmittel, die vollständig, insbesondere ohne gesundheitsschädliche oder reizende Rückstände, entfernt werden können.

Ein weiterer Gegenstand der vorliegenden Erfindung ist ein Verfahren zur Herstellung eines erfindungsgemäßen immunologisch aktiven Phyto-Gemisches umfassend mindestens einen ethanolischen Pflanzenextrakt ausgewählt jeweils aus den erfindungsgemäßen Spezies des Genus a) Bid. alba, Bid. pilosa, und mindestens einen weiteren ethanolischen Pflanzenextrakt ausgewählt aus b) Sta. jamaicensis, Sta. Cayennensis, Sta. indica, und/oder c) Bur. simaruba, Bur. microphylla und/oder Bur. glabrifolia und optional d) Stemodia und/oder Aloe, umfassend die Schritte

- Bereitstellen mindestens eines Pflanzenbestandteils mindestens einer Pflanze ausgewählt aus a) und einer weiteren Pflanze ausgewählt aus b) und/oder c) jeweils ausgewählt aus ober- und/oder unterirdischen Pflanzenbestandteilen, insbesondere Rinde, Stängel, Zweige, Äste, Blätter, Blüte, Knospen, Samen, Hüllen, Pollen, Fruchtorgane, photosynthetisch aktive Bestandteile, Speicherorgane und/oder Wurzel,
- jeweils mindestens einen Extraktionsschritt, wobei als Lösungsmittel wässriger Ethanol mit größer gleich 70 % Ethanol bis kleiner gleich 100 % Ethanol eingesetzt wird, insbesondere mindestens einen Zyklus des Verfahrens umfassend mindestens einen Extraktionsschritt, mindestens einen Trennschritt und optional mindestens einen Trocknungsschritt, und
- Erhalten mindestens eines ethanolischen Pflanzenextraktes der jeweiligen Pflanze, vorzugsweise in trockener oder flüssiger Form, und
- optional einen weiteren Verarbeitungsschritt umfassend Trocknen, Zerkleinerung, Mahlen mittels einer Mühle, Verarbeitung in einem Mörser, Dispergieren, und/oder
- Mischen mindestens zwei der ethanolischen Pflanzenextrakte, wobei einer ausgewählt wird aus a) und der mindestens zweite aus b) und/oder c),
- optional Hinzumischen eines dritten Pflanzenextraktes ausgewählt aus b), c) und/oder d) und

- optional Verarbeitung zu einer homogenen Mischung.

Im Trennschritt wird der erhaltene Auszug mit dem Lösungsmittel von den verbliebenen Pflanzenbestandteilen abgetrennt, vorzugsweise durch Filtration, Dünnschichtchromatographie, Destillation, Ausschütteln oder Evaporation. Die Wahl des Trennschrittes ist Fachwissen des Fachmanns.

In einer Ausführungsform des erfindungsgemäßen Verfahrens werden die mindestens zwei Pflanzenextrakte in flüssiger Form, in trockener Form oder als Gemisch aus festen und flüssigen Formen erhalten. In einer besonderen Ausführungsform werden die mindestens zwei Pflanzenextrakte durch Trocknen der ethanolischen Extrakte und Zugabe eines wässrigen Lösungsmittels in flüssiger Form erhalten.

Die verbliebenen und abgetrennten Pflanzenbestandteile können anschließend einer erneuten Extraktion zugeführt werden und bei Bedarf vorher zerkleinert werden. Die Schritte Extraktion, Trennen, optional Trocknen des erhaltenen Auszugs und Zerkleinern der abgetrennten Pflanzenbestandteile können variabel kombiniert, mit frischen Pflanzenbestandteilen zusammengeführt und wiederholt werden. Die Ausgestaltung des Verfahrens ist Fachwissen des Fachmanns, sodass eine maximale Ausbeute der bereits oben beschriebenen Inhaltsstoffe im erfindungsgemäßen Pflanzenextrakt a) und b) und/oder c) und optional d) und dem erfindungsgemäßen Phyto-Gemisch erzielt wird.

Bevorzugt werden Pflanzenbestandteile der Pflanzen eingesetzt, ausgewählt aus

a) Bid. alba,
b) Sta. jamaicensis und
c) Bur. simaruba und optional
d) dem Genus Aloe und/oder Genus Stemodia, vorzugsweise Stem. maritima, Aloe vera, Aloe barbadensis, Aloe perfoliata, Aloe vulgaris, Aloe indica und/oder Aloe chinensis.

Ein Zyklus des Verfahrens umfasst mindestens einen Extraktionsschritt und mindestens einen Trennschritt. Optional kann zwischen den Zyklen mindestens ein Trocknungsschritt des bereits erhaltenen Auszugs erfolgen. Ein Trockenschritt der abgetrennten Pflanzenbestandteile kann bei Bedarf erfolgen. Vorzugsweise erfolgt bei Verwendung eines toxischen Lösungsmittels ein weiterer Schritt zur Entfernung des Lösungsmittels.

In einer Ausführungsform des erfindungsgemäßen Verfahrens werden unerwünschte Inhaltstoffe des jeweiligen erfindungsgemäßen Pflanzenextraktes a) und b) und/oder c) und optional d) entfernt. Vorzugsweise werden für orale Formulierungen zur Aufnahme über den Magen-Darm-Trakt abführend wirkende Verbindungen, insbesondere Anthrachinone, entfernt.

Bevorzugt erfolgt der mindestens eine weitere Verarbeitungsschritt zur Homogenisierung des erhaltenen flüssigen oder trockenen Pflanzenextraktes vor Mischung der mindestens zwei Pflanzenextrakte und vor der Formulierung zu einer der hierin beschriebenen Verabreichungsform (Beispiel 8a-b) des erfindungsgemäßen Phyto-Gemisches.

Im erfindungsgemäßen Verfahren zur Herstellung eines immunologisch aktiven Phyto-Gemisches umfassend mindestens einen ethanolischen erfindungsgemäßen Pflanzenextrakt a) und mindestens einen weiteren ethanolischen Pflanzenextrakt ausgewählt aus b) und/oder c) und optional d), erfolgt die Extraktion mit mindestens einem Lösungsmittel, ausgewählt aus wässrigem Ethanol, Ethanol, größer gleich 70 % Ethanol bis kleiner gleich 100 % Ethanol. Als Lösungsmittel werden beschrieben Wasser, Methanol, Aceton, Chloroform, n-Butanol, Hexan, Essigsäureethylester und/oder Diethylether, insbesondere einem Gemisch aus mindestens zwei der vorgenannten Lösungsmittel.

Bevorzugt wird als Lösungsmittel wässriger Ethanol größer gleich 70 % Ethanol bis zu kleiner gleich 100 % Ethanol zur Extraktion eingesetzt. Ethanolextrakte der erfindungsgemäßen Spezies a) und b) und/oder c) und optional d) werden aufgrund der geringen bis keinen Toxizität des Ethanols im Gegensatz zu z.B. Methanol, Aceton oder Chloroform, bevorzugt. Nach Trocknung beträgt der Rest-Ethanolgehalt vorzugsweise kleiner gleich 5 %, besonders bevorzugt kleiner gleich 1 %, kleiner gleich 0,1 % an Ethanol. Bevorzugte Lösungsmittel sind wässriger Ethanol, Ethanol, vorzugsweise größer gleich 70 % bis kleiner gleich 100 % Ethanol. Insbesondere größer gleich 75 %, größer gleich 80 %, größer gleich 85 %, größer gleich 90 %, größer gleich 95 %, 96 %, 97 %, 98 % bis kleiner gleich 100 % Ethanol. Besonders bevorzugt wird hochreiner oder biobasierter hochreiner Ethanol verwendet.

In bevorzugter Ausgestaltung des erfindungsgemäßen Verfahrens zur Herstellung eines immunologisch aktiven Phyto-Gemisches im Sinne der Erfindung wird der mindestens eine ethanolische Pflanzenextrakt a) und mindestens ein weiterer ethanolische Pflanzenextrakt ausgewählt aus b) und/oder c) und optional d) in flüssiger Form, in trockener Form oder als Gemisch aus festen und flüssigen Formen erhalten.

"Gemisch" im Zusammenhang mit dem erhaltenen Produkt gemäß dem erfindungsgemäßen Verfahren umfasst trockene und flüssige aus den Pflanzenbestandteilen extrahierte Bestandteile. Flüssige Bestandteile können eine Restfeuchte der aus den Pflanzen stammenden Flüssigkeiten, ölige Bestandteile der Pflanze und Restlösungsmittel umfassen.

Im erfindungsgemäße Verfahren werden die erfindungsgemäßen Pflanzenextrakte ausgewählt aus a), b) und/oder c) insbesondere in flüssiger Form, in trockener Form und/oder als Gemisch aus festen und flüssigen Formen, im jeweiligen Mischschritt gemischt. Insbesondere kann ein Schritt zur Homogenisierung, Dispergierung des mindestens einen Pflanzenextraktes vor dem Mischen durchgeführt werden.

In einer Ausgestaltung des erfindungsgemäßen Verfahrens ist es möglich eine getrocknete Form mindestens eines Pflanzenextraktes, z.B. ein Pulver, mit einer flüssigen Form mindestens eines weiteren Pflanzenextraktes zu mischen. Die flüssige Form kann z.B. ein nicht getrockneter Ethanol-Extrakt sein. Dies hat den Vorteil, dass die Konzentration des Gesamt-Pflanzenextraktes pro Volumen nicht durch zusätzliche Flüssigkeiten gesenkt wird. Darüber hinaus wird die Reinheit des Extraktes verbessert, da Mischungen von Lösungsmitteln vermieden und definierte Konzentrationen und Mischungen hergestellt werden.

Bevorzugt werden trockene Formen der Extrakte zu einem immunologisch aktiven Phyto-Gemisch umfassend mindestens einen erfindungsgemäßen Pflanzenextrakt a) und mindestens einen weiteren ethanolischen Pflanzenextrakt ausgewählt aus b) und/oder c) gemischt und anschließend mit einem ethanolischen Lösungsmittel, vorzugsweise hochreinem Ethanol, zu einer flüssigen Form, vorzugsweise Lösung, Lösung zur Verwendung als Tinktur, Tinktur, Suspension und/oder Dispersion, verarbeitet. Bevorzugt wird ein erfindungsgemäßes Phyto-Gemisch aus trockenen Extrakten gemäß des erfindungsgemäßen Verfahrens erhalten, umfassend

- Bereitstellen mindestens eines Pflanzenbestandteils jeweils von

  a) Bidens alba und/oder Bidens pilosa,
  b) Sta. jamaicensis, Sta. cayennensis und/oder Sta. indica,
  c) Bur. simaruba, Bur. microphylla und/oder Bur. glabrifolia und optional
  d) einer Aloe-Spezies ausgewählt aus Aloe vera, Aloe barbadensis, Aloe perfoliata, Aloe vulgaris, Aloe indica und Aloe chinensis,

- jeweils Extraktion der vorgenannten Pflanzenbestandteile von a) und b) und/oder c) und optional d) mit einem beschriebenen, ethanolischen Lösungsmittel, insbesondere mindestens einen Extraktionsschritt und vorzugsweise jeweils mindestens einen Zyklus des Verfahrens,
- Erhalten jeweils eines flüssigen Pflanzenextraktes von a) und b) und/oder c) und optional d) umfassend mindestens ein ethanolisches Lösungsmittel, und
- Trocknen des jeweils erhaltenen Pflanzenextraktes, vorzugsweise durch Gefriertrocknung,
- optional Wiederholung des Extraktionsschrittes der verbliebenen Pflanzenbestandteile
- optional einen weiteren Verarbeitungsschritt umfassend Trocknen, Zerkleinern, Mahlen mittels einer Mühle, Verarbeiten in einem Mörser, Dispergieren, und
- Mischen der erhaltenen trockenen, vorzugsweise feinen, Pflanzenextrakte a) und b) und/oder c) und optional d),
- Erhalten eines immunologisch aktiven Phyto-Gemisches in trockener Form, und
- Hinzufügen größer gleich 70 % bis kleiner gleich 100 % Ethanol, vorzugsweise größer gleich 75 %, größer gleich 80 %, größer gleich 85 %, größer gleich 90 %, größer gleich 95 %, 96 %, 97 %, 98 % bis kleiner gleich 100 % Ethanol, besonders bevorzugt hochreines Ethanol oder biobasiertes hochreines Ethanol, und
- Erhalten einer mindestens 70 %-igen ethanolischen Lösung, insbesondere Suspension, Tinktur oder Dispersion, bezogen auf 100 ml Gesamtvolumen (ad 100 %), (Beispiel 8b).

In einer Alternative des oben beschriebenen Verfahrens wird die Extraktion mit einem ethanolischen Lösungsmittel durchgeführt und dieses anschließend entfernt und der Pflanzenextrakt getrocknet. Zur Herstellung einer Lösung, Dispersion, Suspension oder Tinktur wird der getrocknete und lösungsmittelfreie Pflanzenextrakt nicht in Ethanol, sondern in einem wässrigen Lösungsmittel ohne Ethanol homogen gemischt. Dies hat den Vorteil, dass bei Anwendung auf der Haut und Schleimhaut, insbesondere auf Wunden, keine reizenden Lösungsmittel enthalten sind. Die "wässrige Alternative" gilt für alle erfindungsgemäßen Formulierungen und Indikationen entsprechend. Insbesondere für Formulierungen zur Anwendung im Auge, auf Nasen- und Mundschleimhaut

"Wässrige Lösungsmittel" bedeutet physiologische, pharmazeutisch verträgliche Lösungen auf Basis von Wasser oder physiologisch verträglicher Puffer-Systeme, in denen die trockenen Pflanzenextrakte homogen zu einer Dispersion oder Lösung homogen mischbar sind. Solche "wässrigen Lösungsmittel" sind dem Fachmann bekannt.

Bevorzugt werden Pflanzenextrakte gemischt, welche gemäß dem erfindungsgemäßen Verfahren erhalten werden aus Pflanzenbestandteilen der Pflanzen ausgewählt aus

  a) Bid. alba oder Bid. pilosa,
  b) Sta. jamaicensis, Sta. cayennensis oder Sta. indica und
  c) Bur. simaruba, Bur. microphylla oder Bur. glabrifolia und optional
  d) mindestens einer Spezies des Genus Aloe und/oder Stemodia, vorzugsweise Stem. maritima, Aloe vera, Aloe barbadensis, Aloe perfoliata, Aloe vulgaris, Aloe indica und/oder Aloe chinensis.

Ein weiterer Gegenstand der vorliegenden Erfindung ist ein immunologisch aktives Phyto-Gemisch erhalten nach dem vorbeschriebenen Verfahren, wobei das Phyto-Gemisch mindestens einen ethanolischen Pflanzenextrakt umfasst, ausgewählt aus a) Bid. alba, Bid. pilosa, und mindestens einen weiteren ethanolischen Pflanzenextrakt ausgewählt aus b) Sta. jamaicensis, Sta. cayennensis, Sta. indica, c) Bur. simaruba, Bur. microphylla und/oder Bur. glabrifolia und optional d) mindestens einen weiteren Extrakt einer weiteren biologisch aktiven Pflanze, vorzugsweise des Genus Aloe und/oder Stemodia, besonders bevorzugt Stem. maritima und/oder mindestens einer Aloe-Spezies.

Die Ausführungen zu den Inhaltstoffen, anti-entzündlichen und antimikrobiellen, vorzugsweise antibakteriellen, Wirksamkeiten gelten für das erfindungsgemäße Verfahren und mittels des Verfahrens unmittelbar erhaltener Erzeugnisse entsprechend. Somit weisen gemäß dem beschriebenen Verfahren erhaltene Erzeugnisse die vorbeschriebenen Wirksamkeiten auf, wie den Beispielen 2 bis 9 zu entnehmen ist.

Ein weiterer Gegenstand der vorliegenden Erfindung ist ein immunologisch aktives Phyto-Gemisch der vorbeschriebenen Art und/oder hergestellt gemäß des vorgeschriebenen Verfahrens umfassend mindestens einen ethanolischen Pflanzenextrakt ausgewählt aus a) Bidens, und mindestens einen weiteren Pflanzenextrakt ausgewählt aus b) Stachytarpheta und/oder c) Bursera und optional d) mindestens einen weiteren Extrakt einer weiteren biologisch aktiven Pflanze zur Verwendung als Arzneimittel, Medizinprodukt, Nahrungsergänzungsmittel, Kosmetikum und/oder als immunologisch aktiver Zusatz zu einem der vorgenannten Erzeugnisse.

Alle erfindungsgemäß beschriebenen Formulierungen eignen sich als oder zur Herstellung von Arzneimitteln, Medizinprodukten, Nahrungsergänzungsmitteln und Kosmetika.

"Nahrungsergänzungsmittel" entsprechen erfindungsgemäß vorzugsweise der EU-Richtlinie 2002/46/EG und umfassen Erzeugnisse zur erhöhten Versorgung des menschlichen Körpers, vorzugsweise als Pulver, Tablette oder Tee jeweils enthaltend das immunologisch aktive Phyto-Gemisch umfassend mindestens einen erfindungsgemäßen Pflanzenextrakt a) und mindestens einen weiteren ethanolischen Pflanzenextrakt ausgewählt aus b) und/oder c) und optional d) oder eine bevorzugte Ausführung des Phyto-Gemisches.

"Medizinprodukte" im Sinne der Erfindung sind die hierin beschriebenen Formulierungen enthaltend das beschriebene immunologisch aktive Phyto-Gemisch umfassend mindestens einen erfindungsgemäßen Pflanzenextrakt a) und mindestens einen weiteren ethanolischen Pflanzenextrakt ausgewählt aus b) und/oder c) und optional d) zur Anwendung bei der Prävention oder Behandlung des Menschen für medizinisch therapeutische Zwecke. Bevorzugt entsprechen die erfindungsgemäßen Medizinprodukte der Richtlinie 93/42/EWG. Beispiele sind Medizinaltee, Tropfen, Tinktur, Salbe, Creme und Paste enthaltend das Phyto-Gemisch.

"Kosmetikum" im Sinne der Erfindung sind topische Kosmetikprodukte zum Aufbringen auf die Haut umfassend das erfindungsgemäße Phyto-Gemisch enthaltend mindestens einen erfindungsgemäßen Pflanzenextrakt a) und mindestens einen weiteren ethanolischen Pflanzenextrakt ausgewählt aus b) und/oder c) und optional d). Solche Kosmetikprodukte sind Creme, Seife, Peeling, Maske, Reinigungslotion oder -milch und verwandte Hautpflegeprodukte.

In einer besonderen Ausführungsform liegt das erfindungsgemäße immunologisch aktive Phyto-Gemisch zur topischen und oralen Verabreichung in wässriger Form vor.

Somit ist ein weiterer Gegenstand eine pharmazeutische oder kosmetische Zusammensetzung umfassend das erfindungsgemäße immunologisch aktive Phyto-Gemisch, das mindestens einen ethanolischen erfindungsgemäßen Pflanzenextrakt umfasst ausgewählt aus a) und mindestens einen weiteren ethanolischen Pflanzenextrakt ausgewählt aus b) und/oder c) und optional d) mindestens einen weiteren Extrakt einer weiteren biologisch aktiven Pflanze umfassend Aloe-Spezies des Genus Aloe der Unterfamilie Asphodeloideae, Spezies des Genus Stemodia der Familie Plantaginaceae und/oder Stem. maritima.

Vorzugsweise enthalten die vorgenannten Zusammensetzungen mindestens einen kosmetisch und/oder pharmazeutisch zulässigen Hilfsstoff. Die beschriebenen bevorzugten Ausführungsformen des Phyto-Gemisches und Formulierungen gelten für die pharmazeutischen oder kosmetischen Zusammensetzungen entsprechend.

Ein weiterer Gegenstand der vorliegenden Erfindung ist ein immunologisch aktives Phyto-Gemisch, vorzugsweise eine Zusammensetzung enthaltend das Phyto-Gemisch, zur Anwendung bei der Prävention oder in einem Verfahren zur Behandlung von Effloreszenzen enthaltend mindestens einen erfindungsgemäßen ethanolischen Pflanzenextrakt ausgewählt aus

a) Bid. alba, Bid. pilosa der Familie Asteraceae, und mindestens einen weiteren ethanolischen Pflanzenextrakt ausgewählt aus

b) Sta. jamaicensis, Sta. cayennensis, Sta. indica und/oder

c) Bur. simaruba, Bur. microphylla und/oder Bur. glabrifolia und optional

d) dem Genus Aloe der Unterfamilie Asphodeloideae, dem Genus Stemodia der Familie Plantaginaceae, vorzugsweise Stemodia maritima, Aloe vera, Aloe barbadensis, Aloe perfoliata, Aloe vulgaris, Aloe indica und/oder Aloe chinensis.

"Effloreszenzen" sind im allgemeinen Hautveränderungen und umfassen kosmetische Effloreszenzen, die ohne gesundheitsschädliche Beeinträchtigung des Menschen auftreten, aber für das psychologische Wohlbefinden des Menschen relevant sind und physiologisch pathologische Effloreszenzen, die zu gesundheitsschädlichen Beeinträchtigungen bis hin zu schweren und lebensbedrohlichen Erkrankungen, wie Hautkrebs, beim Menschen und Tieren führen.

Kosmetische Effloreszenzen umfassen oberflächige Kratzer in der Haut, insbesondere Oberhaut, Reizungen oder Schürfungen der Hautoberfläche, Einreißen der Haut und Rauigkeit jeweils der Haut, insbesondere Oberhaut.

Pathologische Effloreszenzen umfassen Hauterkrankungen, die mit einer Entzündungsreaktion mindestens eines Gewebes der Haut einhergehen und/oder eine mikrobielle, insbesondere bakterielle, Infektion umfassen. Unter pathologischen Effloreszenzen werden auch solche Hautveränderungen zusammengefasst, die ursprünglich als kosmetische Effloreszenzen auftreten und sich im Krankheitsverlauf, insbesondere durch Veränderungen der Konstitution der Hautzellen, zu pathologischen Effloreszenzen entwickeln.

"Effloreszenzen" umfassen erfindungsgemäß Reizungen und Entzündungen der Haut und Schleimhaut, bakterielle Infektionen, mit Virusinfektionen einhergehende bakterielle Infektionen, Mykosen, Folgezustände und Folgeinfektionen von infektiösen und parasitären Krankheiten, Krankheiten des äußeren Ohres, Otitis externa (H60., H62.1, H62.2 ICD-10-GM), Abszess des äußeren Ohres (H60.0 ICD-10-GM), Furunkel des äußeren Ohres, lokale Infektionen der Haut und Unterhaut, Impetigo (L01.- ICD-10-GM), Hautabszesse, Furunkel und Karbunkel (L02.-, L02.0 bis L02.9 ICD-10-GM), mit Pilondialzyste (L05.-) einhergehende Entzündungen der Haut, Erythrasma (L08.1 ICD-10-GM), Bullöse Dermatosen (L10-L14), Dermatitis und Ekzeme (L20-L30), Windeldermatitis (L22), Allergische Kontaktdermatitis (L23), toxische Dermatitis (L24), Pruritus (L29.-), sonstige Dermatitis (L30- bis L30.09), Lichen ruber (L43.-), Papulosquamöse Hautkrankheiten (L45), Urtikaria und Erythem (L50-54), Symptome, die die Haut und das Unterhautgewebe betreffen, Verbrennungen, Verätzungen, Krankheiten der Haut und Unterhaut durch Strahleneinwirkung (L55-L59) einschließlich Sonnenbrand, Dermatitis solaris acuta 1. bis 2. Grades (L55), Erfrierungen, Komplikationen durch medizinische und chirurgische Behandlungen und damit einhergehende Beeinträchtigungen umfassend Wundheilung, Narben und Wunden.

Die Abkürzung "ICD-10-GM" steht für "Internationale statistische Klassifikation der Krankheiten und verwandter Gesundheitsprobleme 10. Revision German Modification" (ICD-10-GM) und wird regelmäßig aktualisiert. Hiermit wird explizit auf diese Klassifikation und die Beschreibung der Hautkrankheiten Bezug genommen und insbesondere der Inhalt des Kapitels XII, L00-L59, in die Offenbarung der vorliegenden Erfindung einbezogen.

"Haut" im Sinne der Erfindung umfasst die Haut der Gliedmaßen, der Extremitäten, der Gelenkbeugen, des Oberkörpers, des Kopfes, des Außenohr, Nase, Nasenschleimhaut sowie Lippen und Mundschleimhaut und die Haut des Auges, insbesondere die Hornhaut, Bindehaut und Schleimhaut des Auges. Insbesondere umfasst "Haut" mindestens eine Gewebeschicht umfassend Stratum corneum, Stratum lucidum, Stratum granulosum, Stratum spinosum, Stratum basale und/oder Stratum germinativum (= Stratum spinosum + Stratum basale), optional der Dermis umfassend Stratum papillare und Stratum reticulare und/oder der Subcutis umfassend Bindegewebe mit Fibroblasten, Endothelzellen, Kollagen und Fettzellen.

Bevorzugt wird das Immunologisch aktive Phyto-Gemisch, vorzugsweise die Zusammensetzung enthaltend das Phyto-Gemisch, zur Anwendung bei der Prävention oder in einem Verfahren zur Behandlung der vorgenannten Effloreszenzen eingesetzt, wobei der mindestens eine ethanolischen Pflanzenextrakt ausgewählt wird aus

a) Bid. alba, Bid. pilosa, und mindestens einen weiteren ethanolischen Pflanzenextrakt ausgewählt aus

b) Sta. jamaicensis, Sta. cayennensis, Sta. indica und/oder

c) Bur. simaruba, Bur. microphylla und/oder Bur. glabrifolia und optional

d) einer Aloe-Spezies des Genus Aloe und/oder des Genus Stemodia, vorzugsweise Stem. maritima, Aloe vera, Aloe barbadensis, Aloe perfoliata, Aloe vulgaris, Aloe indica und/oder Aloe chinensis. Bevorzugt wird eine topische Formulierung, wie Tinktur, Lösung, Suspension, Dispersion, Pulver, Salbe, Paste oder Creme (siehe Beispiel 8a-b).

Vorzugsweise umfasst das immunologisch aktive Phyto-Gemisch zur Anwendung bei der Prävention oder in einem Verfahren zur Behandlung von Effloreszenzen, wie oben beschrieben, als d) mindestens einen weiteren Extrakt einer biologisch aktiven Pflanze mindestens eine Aloe-Spezies des Genus Aloe umfassend Aloe vera, Aloe barbadensis, Aloe perfoliata, Aloe vulgaris, Aloe indica oder Aloe chinensis, Aloe vera, eine Spezies des Genus Stemodia und/oder Stemodia maritima.

In einer bevorzugten Ausgestaltung der Anwendung des erfindungsgemäßen immunologisch aktiven Phyto-Gemisches, vorzugsweise der Zusammensetzung enthaltend das Phyto-Gemisch, zur Prävention oder in einem Verfahren zur Behandlung von Effloreszenzen, umfassend Effloreszenzen kosmetische und pathologische Effloreszenzen, mit der transiente Hautflora assoziierte Hauterkrankungen, bakterielle und/oder virale Infektionen der Haut, Furunkulosen, Mykosen, Entzündungsreaktionen der Haut, Impetigo, benigne und maligne Tumorbildung, Dermatosen, Ekzeme, Pruritus, Psoriasis, Akne, Hautreizungen, Hautrötung, symptomatische Effloreszenzen, Verbrennungen, Verätzungen, Erfrierungen, durch Gifte, Arzneimittel, Drogen, Allergene, Strahlung und als Nebenwirkung auftretende Effloreszenzen, durch Bisse und Stiche von Insekten und Parasiten verursachte Reizungen und Entzündungen der Haut. Insbesondere Effloreszenzen gemäß ICD-10-GM.

Die Wirksamkeit des erfindungsgemäßen Phyto-Gemisches bei einigen der Effloreszenzen unter Anwendung der erfindungsgemäßen Formulierungen ist in Beispiel 9 und in Tabelle 7 beschrieben, ohne sich auf diese zu beschränken.

Bevorzugt umfasst das immunologisch aktive Phyto-Gemisch, vorzugsweise die Zusammensetzung enthaltend das Phyto-Gemisch, zur Anwendung bei der Prävention oder in einem Verfahren zur Behandlung von Effloreszenzen im Sinne der Erfindung mindestens einen Hilfsstoff ausgewählt aus Füllstoffe, Zerfallsbeschleuniger, Gleitmittel, Sprengmittel, Schmiermittel, Formtrennmittel, Fließregulatoren, Lösungsmittel, Emulgatoren, Lösungsvermittler, Solubilisatoren, Netzmittel, Salzbildner, Puffer, Gelbildner, Verdickungsmittel, Filmbildner, Bindemittel, Sorptionsmittel, Süßmittel, Färbemittel, Weichmacher, Stabilisatoren, Matrixbildner, Polymere, Säuerungsmittel, Konservierungsmittel, Duftstoffe und/oder retardierende Mittel.

Jede erfindungsgemäße Formulierung (Beispiel 8a und 8b), vorzugsweise der Zusammensetzung, enthaltend das immunologisch aktive Phyto-Gemisch kann aus dem Stand der Technik bekannte Hilfsstoffe aufweisen. Weitere Hilfsstoffe umfassen Trägerstoffe, Konservierungsstoffe, Antioxidantien, Stabilisatoren, Vitamine, Färbemittel, Geruchsverbesserer und Geschmacksstoffe.

Geschmacksstoffe dienen zur Überdeckung eines potentiell unangenehmen Geschmacks des Phyto-Gemisches, insbesondere der Formulierungen enthaltend das vorbeschriebene Phyto-Gemisch. Hierzu eignen sich z.B. Minze, Anis, Fenchel, Menthol, Kümmel, Medizinalhonig, Honig, Agavensaft, Zucker, Zuckerersatz sowie -austauschstoffe, Propolis und andere nach dem Stand der Technik bekannte Geschmacks- und Aromastoffe.

Für kosmetische Formulierungen, vorzugsweise der Zusammensetzung enthaltend das Phyto-Gemisch, enthaltend das immunologisch aktive Phyto-Gemisch aus a) und b) und/oder c) und optional d), wie Salben, Pasten, Cremes und Gele, werden als Hilfsstoffe tierische und pflanzliche Fette, Wachse, Paraffine, Stärke, Traganth, Cellulosederivate, Polyethylenglykole, Silikone, Bentonite, Kieselsäure, Talkum und Zinkoxid oder Gemische aus mindestens zwei der vorgenannten Stoffe eingesetzt.

Hilfsstoffe für Puder oder Sprays enthaltend das immunologisch aktive Phyto-Gemisch aus a) und b) und/oder c) und optional d) umfassen Milchzucker, Talkum, Kieselsäure, Aluminiumhydroxid, Calciumsilikat, Calcium- und Magnesiumcarbonat, Magnesiumoxid, Metallseifen, Cellulosepulver, reine und modifizierte Stärken und Polymer-, Polyamid-Pulver oder Gemische aus mindestens zwei der vorgenannten Stoffe. Sprays und Aerosole können zusätzlich die üblichen Treibmittel, z.B. Chlorfluorkohlenwasserstoffe, Propan/Butan oder Dimethylether, enthalten, wobei vorzugsweise biologisch und pharmazeutisch verträgliche Treibmittel eingesetzt werden. Puder enthalten vorzugsweise Milchzucker, Kieselsäure, Calciumsilicat und/oder mineralische Asche z.B. aus Getreide.

Lösungen, Dispersionen, Suspensionen und Emulsionen enthaltend das immunologisch aktive Phyto-Gemisch aus a) und b) und/oder c) und optional d) können die üblichen Hilfsstoffe umfassen, wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1, 3-Butylglykol, Öle, Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische aus mindestens zwei der vorgenannten Stoffe.

Suspensionen enthaltend das immunologisch aktive Phyto-Gemisch aus a) und b) und/oder c) und optional d) umfassen übliche Hilfsstoffe, wie flüssige Verdünnungsmittel, z. B. Wasser, Ethanol oder Propylenglykol, Suspendiermittel, z.B. ethoxylierte Iso-stearylalkohole, Polyoxyethylensorbitester und Polyoxyethylensorbitanester, mikrokristalline Cellulose, Aluminiummetahydroxid, Bentonit, Agar Agar und Traganth oder Gemische aus mindestens zwei der vorgenannten Stoffe.

Besonders für die tägliche Anwendung sind Seifen enthaltend das immunologisch aktive Phyto-Gemisch aus a) und b) und/oder c) und optional d) geeignet, welche Hilfsstoffe, wie Alkalisalze von Fettsäuren, Salze von Fettsäurehalbestern, Fettsäureeiweisshydrolysaten, Isothionate, Lanolin, Fettalkohol, Pflanzenöle, Pflanzenextrakte, Glycerin, Zucker oder Gemische aus mindestens zwei der vorgenannten Stoffe enthalten.

Bevorzugte Hilfsstoffe für Formulierungen von Medizinprodukten zur Prävention oder Behandlung von Effloreszenzen im Sinne der vorliegenden Erfindung in fester oder flüssiger Form umfassen Lactose, Saccharose, Dextrose, Mannitol, Sorbitol, Stärke, Gelatine, Tragant, Pektin, Cellulose, Methylcellulose, Hydroxypropylmethylcellulose (HPMC), Carboxymethylcellulose-Natrium, Ethylcellulose, Hydroxypropyl-methylcellulosephthalat, Celluloseacetatphthalat, Polyvinyl-pyrrolidon, Polyvinylalkohol, Polyacrylsäure, Polyethylenglycol, Polyethylenoxid, Natrium-dodecylsulfat, Natriumcetylstearylsulfat und/oder Natriumdioctylsulfosuccinat (auch K, Ca-Salze).

Bevorzugte Hilfsstoffe für erfindungsgemäße Tinkturen, Lösungen, Dispersionen und Suspensionen enthaltend das immunologisch aktive Phyto-Gemisch aus a) und b) und/oder c) und optional d) umfassen Dextrose, Mannitol, Tragant, Pektin, Methylcellulose, Hydroxypropylmethylcellulose (HPMC), Carboxymethylcellulose-Natrium, Polyvinyl-pyrrolidon, Polyvinylalkohol, Polyacrylsäure, Polyethylenglycol, Polyethylenoxid, Natriumdodecylsulfat, Natriumcetylstearylsulfat, und/oder Na-dioctylsulfosuccinat (auch K, Ca-salze) und insbesondere für Suspensionen auch Cellulose.

Halbfeste Formen oder Gemische enthaltend das immunologisch aktive Phyto-Gemisch aus a) und b) und/oder c) und optional d) umfassen bevorzugt Hydroxypropylmethylcellulose (HPMC), Carboxymethylcellulose-Natrium, Polyethylenglycol, Polyethylenoxid, Natrium-dodecylsulfat, Natriumcetylstearylsulfat und/oder Pektin.

In einer besonderen Ausgestaltung des erfindungsgemäßen immunologisch aktiven Phyto-Gemisches umfassend mindestens einen ethanolischen Pflanzenextrakt aus a) und mindestens einen weiteren ethanolischen Pflanzenextrakt aus b) und/oder c) und optional d), vorzugsweise der Zusammensetzung enthaltend das Phyto-Gemisch, zur Anwendung bei der Prävention oder in einem Verfahren zur Behandlung von Effloreszenzen im Sinne der Erfindung liegt das erfindungsgemäße Phyto-Gemisch vor als

- orale Formulierung umfassend i) feste Formen, wie Tablette, Pulver, Granulat, Brausetablette, Trockensaft, Dragee, Globuli, Kapsel und Lyophylisat, ii) flüssige Formen, wie Suspension, Lösung, Dispersion, Tinktur, Konzentrat, Tee, oder iii) Gemisch, wie Spray oder
- topische Formulierung umfassend i) feste Formen, wie Pulver, Badezusatz, Sitzbadpulver, ii) flüssige Formen, wie Suspension, Lösung, Dispersion, Tinktur, Tee oder iii) Gemisch, wie Spray, Aerosol, Lotionen, halbfeste Formen umfassend Salben, Cremes und Pasten.

Tabletten im Sinne der Erfindung umfassen einfache Tabletten, Lutsch-, Sublingual- und Buccaltabletten, oral dispersible Tabletten, Lösungs-, Mikro-, Kau- und Brausetabletten, Punkt-, Gerüst- und Mehrschichttabletten, Dragees und Pellets. Bei Auflösung im Mund und Mundschleimhaut liegt der pH-Wert bevorzugt in den beschriebenen pH-Bereichen.

Gemisch im Zusammenhang einer Formulierung (Beispiel 8a und 8b) umfassen halbfeste Formen. Zu den halbfesten Formen von Arzneimitteln und Kosmetikprodukten gehören salbenartige Zubereitungen, die sich dadurch auszeichnen, dass sie nur eine begrenzte Formstabilität aufweisen. Salben und Cremes sind alle streichfähigen Zubereitungen, die auf der Haut oder Schleimhaut appliziert werden. Je nach Beschaffenheit werden halbfeste Formen als Salben, Creme, Pasten oder Gele unterschieden.

Salben im engeren Sinn sind Zubereitungen ohne wässrige Phase. Es werden hydrophobe, wasseraufnehmende und hydrophile Salben unterschieden. Cremes sind Salben, die neben einer Lipidphase eine wässrige Phase enthalten. Pasten sind hochkonzentrierte Suspensionssalben, die physikalisch-chemisch den Übergang von hochkonzentrierten Suspensionen zu feuchten Pulvern darstellen.

Bevorzugt liegt das erfindungsgemäße immunologisch aktive Phyto-Gemisch umfassend mindestens einen erfindungsgemäßen ethanolischen Pflanzenextrakt aus a) und mindestens einen weiteren ethanolischen Pflanzenextrakt ausgewählt aus b) und/oder c) und optional d), vorzugsweise die Zusammensetzung enthaltend das Phyto-Gemisch, zur Anwendung bei der Prävention oder in einem Verfahren zur Behandlung von Effloreszenzen im Sinne der Erfindung als Tinktur, insbesondere als Lösung, Suspension, Dispersion oder in halbfester Form, vor (Beispiel 9), umfassend

- größer gleich 1 Gew.-%, vorzugsweise bis kleiner gleich 50 Gew.-%, bevorzugt größer gleich 3 Gew.-%, größer gleich 5 Gew.-%, größer gleich 7 Gew.-%, bezogen auf den Gesamtgehalt der Tinktur (T = 100 Gew.-%), mindestens eines Pflanzenextraktes ausgewählt aus Bid. alba, Bid. pilosa, und mindestens einen weiteren Pflanzenextrakt ausgewählt aus Sta. jamaicensis, Sta. cayennensis, Sta. indica und/oder Bur. simaruba, Bur. microphylla, Bur. glabrifolia.
- mindestens ein Säuerungsmittel ausgewählt aus Essigsäure, Citronensäure, Ascorbinsäure, Adipinsäure, Weinsäure, Mandelsäure und/oder Apfelsäure,
- größer gleich 1 Gew.-%, vorzugsweise größer gleich 3 Gew.-%, größer gleich 5 Gew.-%, größer gleich 7 Gew.-%, größer gleich 10 Gew.-%, bis jeweils kleiner gleich 70 Gew.-%, eines d) Aloe-Extrakts, bezogen auf den Gesamtgehalt der Tinktur (T = 100 Gew.-%), und
- mindestens einen Hilfsstoff,

wobei die Tinktur einen pH-Wert größer gleich 3 bis kleiner gleich 9 aufweist und die Tinktur ein wässrig/ethanolisches Gemisch ist, insbesondere eine Lösung oder Dispersion, mit einer Ethanolkonzentration größer gleich 70 %, bezogen auf 100 ml Gesamtvolumen der Tinktur (ad 100 %). Bevorzugt enthält die vorbeschriebene Tinktur größer gleich 3 Gew.-%, größer gleich 5 Gew.-%, vorzugsweise größer gleich 7 Gew.-%, Gesamtgehalt an mindestens einem erfindungsgemäßen Pflanzenextrakt oder Kombination aus a) und mindestens einem weiteren ethanolischen Pflanzenextrakt ausgewählt aus b) und/oder c) und vorzugweise größer gleich 15 Gew-% mindestens eines d) Aloe-Extraktes, jeweils bezogen auf den Gesamtgehalt des Phyto-Gemisches (ad 100 Gew.-%).

Die vorbeschriebene Tinktur, Lösung, Suspension, Dispersion und halbfeste Formen entsprechend, weist abhängig von Ihrer jeweiligen Indikation bevorzugte pH-Werte auf, die bereits oben detailliert im Zusammenhang mit den Säuerungsmitteln beschrieben wurden.

"Wässrig/ethanolisches Gemisch" im Sinne der Erfindung umfasst Lösungen und Dispersionen enthaltend unlösliche Bestandteile und Partikel. Das Vorliegen des wässrig/ethanolischen Gemisches ist abhängig von dem Lösungsverhalten der extrahierten Bestandteile, des Trennschrittes und abhängig von der Ethanolkonzentration.

Ebenfalls bevorzugt ist das erfindungsgemäße immunologisch aktive Phyto-Gemisch umfassend mindestens einen erfindungsgemäßen Pflanzenextrakt aus a) und mindestens einen weiteren ethanolischen Pflanzenextrakt ausgewählt aus b) und/oder c) und optional d), vorzugsweise die Zusammensetzung enthaltend das Phyto-Gemisch, zur Anwendung bei der Prävention oder in einem Verfahren zur Behandlung von Effloreszenzen im Sinne der Erfindung, wobei die orale Formulierung (siehe Beispiel 8a-b und 9) auf einer festen Form basiert, wobei

- die feste Form mindestens einen trockenen, insbesondere lösungsmittelfreien, Pflanzenextrakt umfasst, ausgewählt aus Bid. alba, Bid. pilosa, und mindestens einen weiteren Pflanzenextrakt ausgewählt aus Sta. jamaicensis, Sta. cayennensis, Sta. indica, Bur. simaruba und/oder einer Aloe-Spezies,
- der mindestens eine Pflanzenextrakt mit einem Gehalt größer gleich 1 Gew.-%, vorzugsweise größer gleich 3 Gew.-%, größer gleich 5 Gew.-%, größer gleich 7 Gew.-% bezogen auf den Gesamtgehalt der festen Form (F = 100 Gew.-%) enthalten ist,
- die Restfeuchte kleiner gleich 5 Gew.-% beträgt, vorzugsweise kleiner gleich 1 Gew.-% und besonders bevorzugt kleiner gleich 0,1 Gew.-% ist, bezogen auf den Gesamtgehalt des trockenen Pflanzenextraktes (F = 100 Gew.-%) und
- mindestens ein Hilfsstoff enthalten ist.

Besonders bevorzugte topische Formulierungen sind solche zum Auftragen, Aufbringen, Einmassieren, Aufsprühen, Auftropfen, und/oder Auflegen auf die betroffene Hautoberfläche und damit zur Aufnahme des immunologisch aktiven Phyto-Gemisches über die Haut oder Schleimhaut, insbesondere Nasen- und/oder Mundschleimhaut.

Besonders bevorzugte orale Verabreichungsformen des erfindungsgemäßen Phyto-Gemisches umfassend mindestens einen erfindungsgemäßen Pflanzenextrakt a) und mindestens einen weiteren ethanolischen Pflanzenextrakt ausgewählt aus b) und/oder c) und optional d) umfassen Formulierungen zum Lösen in einem Getränk und anschließendem Trinken, zum Schlucken (z.B. als Tablette) und Aufnahme über den Magen-Darmtrakt, zur oralen Aufnahme und Zerfall im Mundraum oder Aufbringen auf die Mundschleimhaut und Aufnahme über die Mundschleimhaut. Formulierungen zur Aufnahme über den Magen-Darm-Trakt enthalten bevorzugt keine Anthrachinone, da diese eine abführende Wirkung haben können. Erfindungsgemäße Formulierungen für Indikationen, bei denen eine abführende Wirkung unerwünscht ist, enthalten vorzugsweise keinen Aloe-Extrakt oder einen Anthrachinon-freien Aloe-Extrakt. Entsprechendes gilt für die erfindungsgemäßen Spezies des Genus a) Bidens, b) Stachytarpheta und c) Bursera.

Die nachfolgenden Beispiele erläutern die Erfindung näher und stellen lediglich ausgewählte Ausführungsformen dar ohne sich darauf zu beschränken. Hierzu wurden einzelne erfindungsgemäße Spezies als Vertreter des jeweiligen Genus a) Bidens, b) Stachytarpheta und c) Bursera und für die als bevorzugt beschriebenen Spezies des jeweiligen Genus eingesetzt.

**Beispiele**

**Beispiel 1: Ursprung des verwendeten Pflanzenmaterials**

Die im Rahmen dieser Patentanmeldung offenbarten und beanspruchten Pflanzen Bur. simaruba, Sta. jamaicensis, Bid. alba und Stem. maritima wurden jeweils als erfindungsgemäße Vertreter des Genus a) Bidens, b) Stachytarpheta, c) Bursera und d) Stemodia von den Bahamas, Insel "Long Island", bezogen. Für die Ernte der hier verwendeten Pflanzen und dessen Export nach Deutschland für Forschungs- und Versuchszwecke erließ die Regierung der Bahamas entsprechende Zertifikate ("Plant Protection Service of the Bahamas" Nr. 2928 und Nr. 2929 und "Permit to conduct Scientific Research in the Bahamas"). Im Rahmen dieser Forschungserlaubnis und Ausfuhrgenehmigung wurden die nachfolgend beschriebenen Versuche durchgeführt.

**A: Bidens alba (Bid. alba) / B: Stachytarpheta jamaicensis (Sta. jamaicensis)**

Von jungen 1 bis 3-jährige Pflanzen (A und B) wurden jeweils oberirdische Pflanzenbestandteile umfassend Stängel, Blätter, Triebe und Blüten frisch geerntet. Diese wurden bei 40 °C bis 55 °C für 6 bis 20 Stunden getrocknet und anschließend kühl und trocken gelagert. Die Dauer der Trocknung und Temperatur ist variabel nach dem Fachwissen des Fachmanns frei wählbar.

**C: Bursera simaruba (Bur. simaruba)**

Blätter und Äste eines 5 bis 20-jährigen Baumes. Die geernteten Blätter wurden bei 57 °C bis 62 °C für 8 bis 20 Stunden getrocknet und anschließend kühl und trocken gelagert.

**D1: Stemodia maritima (Stem. maritima)**

Oberirdisches ca. 1-jähirges Kraut. Das geerntete Kraut wurde bei etwa 40 °C für 7 Stunden getrocknet und anschließend kühl und trocken gelagert.

**D2: Aloe vera**

Ausgangsmaterial: 500 ml eines Anthrachinon-freien Wasserextraktes von Aloe vera (allcura Naturheilmittel GmbH, Reichenäcker 7, 97877 Wertheim). 20 ml des Wasserextraktes wurden gefriergetrocknet (Christ LMC-1, Delta 1-20 KD). **Ausbeute**: Trockengewicht 450 mg Pulver (etwa 22,5 mg/ml).

Tabelle 1b: Pflanzenmaterial

| Pflanze | Pflanzenmaterial als Pulver nach Zerkleinerung [g] | Trockengewicht Wasserextrakt [g] | Trockengewicht Ethanolextrakt [g] |
|---|---|---|---|
| A | 31,5 | 0,931 | 1,464 |
| B | 38,7 | 0,7275 | 2,485 |
| C | 33,1 | 1,1212 | 5,8 |
| D1 | 38,7 | 0,299 | 1,39 |
| D2 | ./. | 0,450 | ./. |

**Beispiel 2: Zerkleinerung des Pflanzenmaterials**

Das vorbeschriebene Pflanzenmaterial wurde mit den beschriebenen Pflanzenbestandteilen bereitgestellt, zerkleinert und für etwa 2 Minuten in einem kommerziellen Mixer pulverisiert. Anschließend wurde das jeweilige Pulver zu einem Teil zu einem wässrigen und zum anderen Teil zu einem ethanolischen Extrakt verarbeitet.

**Beispiel 3: Herstellung eines wässrigen Pflanzenextraktes (Stand der Technik)**

**A Bidens alba** - **Wässriger Extrakt:**

Das Pulver wurde in einen Dreihalskolben mit destilliertem Wasser überführt (8,2 g Pulver auf 120 ml destilliertes Wasser) und bei 40 °C zweimal jeweils für 4 Stunden oder insgesamt 8 Stunden gerührt. Nach anschließender Filtration der Extrakt wurde eine klare Lösung erhalten. Die klare Lösung wurde für 64 Stunden der Gefriertrocknung (Christ LMC-1, Delta 1-20 KD) unterzogen. Ausbeute nach Gefriertrocknung: 0,931 g

**B Stachytarpheta jamaicensis** - **Wässriger Extrakt:**

8,0 g des Pulvers von Stachytarpheta jamaicensis wurden analog zu Bidens alba verarbeitet. Ausbeute nach Gefriertrocknung: 0,7275 g

**C Bursera simaruba** - **Wässriger Extrakt:**

8,2 g des Pulvers von Bursera simaruba wurden analog zu Bidens alba verarbeitet. Ausbeute nach Gefriertrocknung: 1,1212 g

**D1 Stemodia maritima** - **Wässriger Extrakt:**

8,1 g des Pulvers von Stemodia maritima wurden analog zu Bidens alba verarbeitet. Ausbeute nach Gefriertrocknung: 0,299 g

**Beispiel 4: Herstellung eines ethanolischen Pflanzenextraktes (im Sinne der Erfindung)**

**A Bidens alba** - **Ethanolextrakt:**

Das Pulver (8,0 g) wurde in eine Extraktionshülse (Macherey & Nagel; MN 645; 23 x 100 mm) gefüllt und die Extraktion mit Rückfluss für 8 Stunden unter Verwendung einer Soxhlet-Extraktion mit 280 ml 96 % Ethanol bei Raumtemperatur durchgeführt. Die organische Phase wurde über Glaswolle (Firma Migge No. 1408/3) filtriert. Anschließend wurde der Ethanolextrakt unter Verwendung eines Rotavapors (Heidolph; 50°C Wasserbadtemperatur, 112 bar) bis zur Trockenheit evaporiert. Ausbeute: 1,464 g Trockengewicht

Das Trockengewicht wurde in 15 ml Ethanol gelöst, um die am Boden des Rundkolbens haftenden Trockenrückstände abzulösen. Anschließend wurde die Lösung auf Probenbehälter zur Lagerung bei -20 °C aufgeteilt und mittels Durchblasen von Stickstoffgas getrocknet.

**B Stachytarpheta jamaicensis** - **Ethanolextrakt:**

8,0 g des Pulvers von Stachytarpheta jamaicensis wurden analog zu Bidens alba verarbeitet. Ausbeute: 2,485 g Trockengewicht

**C Bursera simaruba** - **Ethanolextrakt:**

8,2 g des Pulvers von Bursera simaruba wurden analog zu Bidens alba verarbeitet. Ausbeute: 5,8 g Trockengewicht

**D1 Stemodia maritima** - **Ethanolextrakt:**

6,7 g des Pulvers von Stemodia maritima wurden analog zu Bidens alba verarbeitet. Ausbeute: 1,39 g Trockengewicht

Der Rest-Ethanolgehalt der obigen Ethanol-Pflanzenextrakte betrug weniger als 0,1 %.

**Beispiel 5: Bestimmung der Zytotoxizität der hergestellten Extrakte**

Zellkultur

HaCaT-Zellen (human in vitro spontan transformierte Keratinozyten aus histologisch normaler Haut) von DSMZ (Deutsche Sammlung von Mikroorganismen und Zellkulturen) wurden in Dulbeccos modifiziertem Eagle Medium (DMEM) mit Glutamax (Invitrogen/Gibco, Karlsruhe, Deutschland) mit 10% fötalem Kälberserum (Sigma Aldrich, Deutschland), 100 U/ml Penicillin und 100 $\mu$g/ml Streptomycin und 1 % NEAA gehalten.

Die Zellen wurden bei 37°C, 5 % $CO_2$ und 95 % Luftfeuchtigkeit kultiviert. Die Zellen wurden subkultiviert durch Entfernen des Mediums, Hinzufügen von 0,075 % EDTA-Lösung in 1-fach PBS (Phosphat-gepufferte Salzlösung) und anschließend 10 min bei 37 °C inkubiert. Das EDTA wurde entfernt und die HaCaT-Zellen wurden durch Zugabe von 0,25 % Trypsin und 0,02 % EDTA für 5 min bei 37 °C von dem Kulturgefäß abgelöst. Dann erfolgte die Zugabe von frischem Medium, Zentrifugation, Absaugen des Mediums und Überführen in einen neue Kolben (Verhältnis 1: 5 oder 1:10). Alle Versuche wurden mit Zellen in der logarithmischen Wachstumsphase durchgeführt.

Probenvorbereitung: Die wässrigen Extrakte wurden in Wasser (100 mg /ml Wasser) und die Ethanolextrakte wurden in Dimethylsulfoxid, DMSO, (200 mg/ml DMSO) gelöst.

MTT-Assay nach Mosmann. 1983 (J. of Immunological Methods 65, 55-63)

Zur Bestimmung der konzentrationsabhängigen Zytotoxizität wurden 2 x 10^4 HaCat-Zellen/Probenvertiefung einer 96-Wellplatte überführt und für 24 Stunden inkubiert. Die Inkubation der HaCat-Zellen erfolgte mit 100 $\mu$l des Medium enthaltend unterschiedliche Konzentrationen des jeweiligen Pflanzenextraktes (siehe Tabelle 2) oder einer 1:1 Mischung aus zwei Pflanzenextrakten (siehe Tabelle 3). Anschließend wurden 0,5 mg/ml MTT (3-(4,5-Dimethylthiazol-2-yl)-2,5-diphenyltetrazoliumbromid) hinzugefügt und für 4 Stunden inkubiert. Nach Lösung der von den HaCat-Zellen gebildeten Formazankristalle in 100 $\mu$l DMSO (10 Minuten Schütteln) wurde die Absorption bei 570 nm gemessen (mittels Tecan Safire II Reader, Tecan Crailsheim, Deutschland). Als Referenz wurde Doxorubicin vermessen. Die Zell-Vitalität wurde bestimmt gemäß folgender Formel:

$$\text{Vitalität [\%]} = \frac{[(\text{OD Probe behandelter HaCat-Zellen}) - (\text{OD Probe Mediumkontrolle})] * 100}{[(\text{OD unbehandelte HaCat-Zellen}) - (\text{OD Mediumkontrolle})]}$$

Statistische Analyse

Es wurde eine Dreifachbestimmung (n=3) mit dreifacher Wiederholung durchgeführt und die Ergebnisse als Mittelwerte $\pm$ Standardabweichung angegeben. Die $IC_{50}$-Werte wurden mittels der Dosis-Wirkungs-Kurve berechnet, die unter Verwendung einer logistischen Regressionskurve mit 4 Parametern mit der GraphPad Prism® 5.01 Software oder SigmaPlot® 11.0 erstellt wurde. Die einzelnen Kurven wurden hier nicht abgebildet. Die ermittelten $IC_{50}$-Werte mit den Standardabweichungen sind in Tabelle 2 und 3 zusammengefasst.

Ergebnisse

Keines der wässrigen Pflanzenextrakte Bid. alba (A-W), Sta. jamaicensis (B-W), Bur. simaruba (C-W), Stem. maritima und Aloe vera weist, verglichen mit Doxorubicin als zytotoxische Referenzverbindung, eine Zytotoxizität auf (siehe Tabelle 2).

Ethanolische Pflanzenextrakte von Bid. alba und Bur. simaruba weisen, verglichen mit Doxorubicin als zytotoxische Referenzverbindung, keine Zytotoxizität auf. Ethanolische Extrakte von Sta. jamaicensis und Stem. maritima weisen eine geringe Zytotoxizität auf.

Von den Kombinationen wurden lediglich die mit Pflanzenextrakten etwas höherer $IC_{50}$-Werte erneut auf Zytotoxizität hin untersucht (siehe Tabelle 3).

**Beispiel 6: Bestimmung der anti-entzündlichen Aktivität**

Die Inhibierung der 5-Lipoxygenase (5-LOX) durch die Einzelextrakte und deren Kombinationen (siehe Tabelle 2 und 3) wurden spektrophotometrisch bestimmt (Baylac & Racine 2003). Hierzu wurden 970 $\mu$l des Phosphat-Puffers (21,2 g $K_3PO_4$ in 1 L $H_2O$), pH 9,0 mit 10 $\mu$l der 1 mg/ml konzentrierten 5-LOX (lyophilisiertes Pulver, Fluka) und 20 $\mu$l unterschiedlicher Konzentrationen (0,48 $\mu$g/ml bis 250 $\mu$g/ml) der Einzelpflanzenextrakte und der Kombinationen (1:1 Mischung) gemischt. Die Mischung wurde bei Raumtemperatur für 10 Minuten inkubiert. Die enzymatische Reaktion wurde durch Hinzufügen von 20 $\mu$l einer 5 mM Natrium-Linoleat-Lösung (Sigma Aldrich) initiiert. Die Reaktionskinetik wurde bei 234 nm alle 5 Sekunden für 1 Minute mittels eines WPA Biowave II Spektrophotometers ermittelt. Als Positivkontrolle eines 5-LOX-selektiven Inhibitors wurde Nordihydroguaiarinsäure (NDGA) eingesetzt.

Die initiale Reaktionsgeschwindigkeit wurde jeweils aus der Steigung des linearen Teils der Kurve bestimmt. Die Inhibierung der Enzymaktivität wurde aus Dreifachexperimenten berechnet. Der Prozentsatz der initialen Aktivität wurde gemäß folgender Formel ermittelt:

$$\text{[\%] der initialen Aktivität} = (IA_{Kontrolle} - IA_{Probe}) / IA_{Kontrolle} \times 100$$

wobei Probe einem Einzel-Pflanzenextrakt oder einer Kombination aus mindestens zwei Pflanzenextrakten entspricht und Kontrolle gleich NDGA.

Die Prozent [%] der initialen Aktivität wurden (Y-Achse) als Funktion der Konzentration des jeweiligen Pflanzenextraktes oder der Kombination (1:1 Mischung) graphisch aufgetragen, um den $IC_{50}$ Wert als die Konzentration zu ermitteln, bei der die Enzymaktivität zu 50 % durch den jeweiligen Pflanzenextrakt oder der Kombination inhibiert wird.

Ergebnisse

Aus Tabelle 2 ist ersichtlich, dass der ethanolische Bur. simaruba (C-E) Extrakt eine signifikante Inhibierung der 5-Lipoxygenase aufweist. Somit weist ein Ethanol-Pflanzenextrakt von Bur. simaruba (C-E mit $IC_{50}$ = 132 $\pm$ 13 $\mu$g/ml) eine nachweislich signifikante antientzündliche Aktivität auf. Entsprechendes gilt für den jeweils ethanolischen Einzelextrakt von Sta. jamaicensis (B-E mit $IC_{50}$ = 84 $\pm$ 10 $\mu$g/ml) und Bid. alba (A-E mit $IC_{50}$ = 139 $\pm$ 7 $\mu$g/ml).

Aus Tabelle 3 ist ersichtlich, dass die Kombinationen der jeweiligen Extrakte eine erhöhte Inhibierung der 5-Lipoxygenase aufweisen. Diese Versuche weisen für Bid. alba, Sta. jamaicensis und Bur. simaruba als Vertreter für den Genus a) Bidens, b) Stachytarpheta und c) Bursera jeweils eine signifikante anti-entzündliche Wirksamkeit nach. Diese Wirksamkeit kann auf die, in den vorgenannten Spezies, enthaltenden und mittels des erfindungsgemäßen Verfahrens extrahierten Verbindungen zurückgeführt werden, umfassend Verbascoside, Flavonoide, Iridoide, Ipolamiide, Fulvoipolamiide, Sesquiterpenlactone und/oder Proazulene.

Tabelle 2: Einzelextrakte (nicht erfindungsgemäß)

| Extrakt | Pflanze | Zytotoxizität IC$_{50}$ [μg/ml] | 5-Lox-Inhibierung IC$_{50}$ [μg/ml] |
|---|---|---|---|
| A-W | Bid. alba | >3000 | >1000 |
| A-E | Bid. alba | 527 ± 68 | **139 ± 7** |
| B-W | Sta. jamaicensis | 1023 ± 153 | >1000 |
| B-E | Sta. jamaicensis | 546 ± 66 | **84 ± 10** |
| C-W | Bur. simaruba | 4670 ± 400 | >1000 |
| C-E | Bur. simaruba | 1800 ± 670 | **132 ± 13** |
| D1-W | Stem. maritime | 1399 ± 321 | >1000 |
| D1-E | Stem. maritima | 127 ± 5 | >1000 |
| D2 | Aloe vera | 3360 ± 860 | >1000 |
| Doxorubicin | | 8,6 ± 2,03 | ./. |
| NDGA | | ./. | 0,53 ± 0,09 |
| W = wässriger Extrakt; E = Ethanolextrakt, A, B, C, D1 und D2 entspricht Nomenklatur aus Beispiel 1 | | | |

Tabelle 3: Extrakt-Kombinationen 1:1 Mischung (erfindunasaemäß: A+B, oder A+C)

| Extrakt-Kombination 1:1 Mischung | Zytotoxizität IC$_{50}$ [μg/ml] | 5-Lox-Ihibierung IC$_{50}$ [μg/ml] |
|---|---|---|
| A-E / B-E | n.b. | **52 ± 1,3** |
| A-E / C-E | n.b. | **78 ± 6** |
| B-E / C-E | n.b. | **57 ± 8** |
| A-E / D1-E | 101 ± 15 | 98 ± 7,5 |
| B-E / D1-E | 117 ± 8,7 | 94 ± 4,5 |
| C-E / D1-E | 144 ± 16 | 99 ± 14 |
| A-E / D2 | n.b. | 115 ± 15 |
| B-E / D2 | n.b. | 73 ± 16 |
| C-E / D2 | 2710 ± 290 | 161 ± 23 |
| D1-E / D2 | 128 ± 13 | 130 ± 18 |
| Doxorubicin | 8,6 ± 2,03 | ./. |
| NDGA | ./. | 0,53 ± 0,09 |
| W = wässriger Extrakt; E = Ethanolextrakt, A, B, C, D1 und D2 entspricht Nomenklatur aus Beispiel 1 | | |

**Beispiel 7: Bestimmung der antimikrobiellen Wirksamkeit**

Die antibakterielle Wirksamkeit der jeweiligen Pflanzenextrakte als Einzelextrakt und als Kombination aus zwei Einzelextrakten wurde gegen die folgenden Testkeime untersucht:

Gram-positive Bakterien

- Methicillin resistente Staphylococcus aureus MRSA NCTC 10442 (MRSA NCTC 10442)
- Staphylococcus aureus ATCC 25923 (S. aureus ATCC 25923)

- Staphylococcus epidermidis ATCC 14990 (S. epid. ATCC 14990)

Gram-negative Bakterien

- Pseudomonas aeruginosa ATCC 27853 (P. aerug. ATCC 27853) und
- Acenitobacter baumanii ATCC BAA747 (ATCC BAA747)

Kulturvorbereitung

Die Bakterien wurden auf Columbia-Medium mit 5 % Schafsblut bei 37 °C für 24 Stunden vorkultiviert. Eine bis zwei Kolonien wurden in Kochsalzlösung (0,9 % NaCl) suspendiert und auf eine Trübung von 0,5 McFarland-Standard eingestellt, welcher $1 \times 10^8$ Kolonie-bildende-Einheiten pro Milliliter (KBE/ml) entspricht. Anschließend wurde auf $1 \times 10^6$ KBE/ml verdünnt.

Bestimmung der minimalen Hemmkonzentration (MIC) und der minimalen bakteriziden Konzentration (MBC)

Mittels des Mikrobouillon-Verdünnungsverfahren nach NCCLS (2006) wurde die MIC bestimmt. Hierzu wurden die wässrigen Extrakte in Wasser (w/v) und die erfindungsgemäßen ethanolischen Extrakte in 5 %-iger DMSO (w/v) mit jeweils 80 mg/ml gelöst und Bur. simaruba sowie Aloe vera mit 160 mg/ml suspendiert. Die Extrakte und die Kombinationen (1:1 Mischung) aus zwei Pflanzenextrakten wurden jeweils in eine Probenvertiefung einer 96-Wellplatte überführt. Die Einzelextrakte wurden jeweils auf Konzentrationen von 4 mg/ml bis 8 mg/ml und 0,03 mg/ml eingestellt und die Kombinationen auf 2:2 mg/ml bis 0,3:0,3 mg/ml. Anschließend wurden jeweils die Bakteriensuspensionen mit etwa $5 \times 10^5$ KBE/ml in Müller Hinton-Medium (Fluka) hinzugefügt. Die entsprechend bestückten 96-Wellplatten wurden für 24 Stunden bei 37 °C inkubiert.

Die MIC wurde jeweils anhand der Trübung bei 600 nm ermittelt. Um die MBC zu ermitteln, wurden 3 µl der Suspension aus der jeweiligen Probenvertiefung auf einem Vollnährmedium ausgestrichen und bei 37 °C für 24 Stunden inkubiert. Die MBC wurde als die geringste Konzentration des jeweiligen Extraktes ermittelt, die die Mikroorganismen vollständig abtötet.

Alle Versuche wurden als Dreifachbestimmung durchgeführt (Tabellen 4 bis 5). Als Positivkontrollen für die antimikrobielle Wirkung wurden die Antibiotika Vancomycin (kurz: Van) und Streptomycin (kurz: Strep) zu jedem Versuchsansatz parallel eingesetzt (Tabelle 5c). Als Negativkontrolle für eine hemmende Wirkung durch das verwendete Lösungsmittel wurde jeweils einer der genannten Testkeime mit dem jeweiligen Lösungsmittel (Wasser, DMSO) mitgeführt. Bei allen Negativkontrollen wurde kein negativer Einfluss auf das Wachstum im Vergleich zur Wachstumskontrolle ohne jedweden Zusatz festgestellt. Eine Sterilitätskontrolle bestätigte die Sterilität der verwendeten Medien (Daten nicht gezeigt). Die Zusammensetzung der Kontrollen ist in Tabelle 5d zusammengefasst.

Ergebnisse

Tabelle 4a fasst die antimikrobielle Aktivität der wässrigen Einzelpflanzenextrakte zusammen. Aus Tabelle 4a ist für Sta. jamaicensis (B-W) und Bid. alba (A-W) eine antibakterielle Wirksamkeit zu entnehmen. Stem. maritima (D1-W) weist eine verglichen zu B-W und A-W höhere antibakterielle Wirksamkeit auf.

Die folgenden Kombinationen (1:1 Mischung) der wässrigen Extrakte zeigten alle dieselbe Wirksamkeit gegen gram-positive Bakterien mit MIC $\geq$ 2/2 und MBC $\geq$ 2/2: A-W / B-W, A-W / C-W, B-W / C-W, C-W / D1-W, B-W / D1-W, A-W / D1-W, A-W / D2, B-W / D2, C-W / D2, D1-W / D2

Tabelle 5a fasst die antimikrobielle Aktivität der ethanolischen Einzelextrakte und Tabelle 5b die erfindungsgemäßen Kombinationen (A+B, oder A+C) der ethanolischen Pflanzenextrakte zusammen. Die Versuche weisen deutlich eine antibakterielle Wirksamkeit von Sta. jamaicensis und Bid. alba sowie von Stem. maritima nach. Insbesondere weisen die vorgenannten Spezies eine signifikante Wirksamkeit gegen gram-positive Bakterien und besonders gegen MRSA auf.

Tabelle 4: Antimikrobielle Aktivität der wässrigen Einzel-Pflanzenextrakte

| Testkeim | MIC | MBC | MIC | MBC | MIC | MBC | MIC | MBC | MIC | MBC | MIC | MBC | MIC | MBC |
|---|---|---|---|---|---|---|---|---|---|---|---|---|---|---|
| | C-W | | B-W | | A-W | | D1-W | | D2 | | Van | | Strep | |
| MRSA NCTC 10442 | >8 | >8 | 4 | >4 | >4 | >4 | 2 | 4 | 8 | >8 | 1 | 2 | ./. | n.b. |
| S. aureus ATCC 25923 | >8 | >8 | 4 | >4 | >4 | >4 | 2 | 4 | 4 | >8 | 0,5 | 0,5 | 2 | 8 |
| S. epid. ATCC 14990 | >8 | >8 | 4 | >4 | >4 | >4 | 2 | 4 | 8 | >8 | 1 | 2 | 1 | 8 |
| | | | | | | | | | | | | | | |
| P. aerug. ATCC 27853 | >8 | >8 | >4 | >4 | >4 | >4 | >4 | >4 | >8 | >8 | ./. | n.b. | 4 | 8 |
| ATCC BAA747 | >8 | >8 | >4 | >4 | >4 | >4 | >4 | >4 | 8 | >8 | 64 | 128 | 2 | 4 |

Tabelle 5a: Antimikrobielle Aktivität der ethanolischen Einzel-Pflanzenextrakte

| Testkeim | MIC | MBC | MIC | MBC | MIC | MBC | MIC | MBC | MIC | MBC | MIC | MBC | MIC | MBC |
|---|---|---|---|---|---|---|---|---|---|---|---|---|---|---|
| | C-E | | B-E | | A-E | | D1-E | | D2 | | Van | | Strep | |
| MRSA NCTC 10442 | >8 | >8 | 2 | 4 | 4 | >4 | 0.5 | 1 | 8 | >8 | 1 | 2 | ./. | n.b. |
| S. aureus ATCC 25923 | >8 | >8 | 1 | 4 | 2 | 4 | 0,5 | 1 | 4 | >8 | 0,5 | 0,5 | 2 | 8 |
| S. epid. ATCC 14990 | >8 | >8 | 1 | 2 | 1 | 2 | 0,5 | 1 | 8 | >8 | 1 | 2 | 1 | 8 |
| | | | | | | | | | | | | | | |
| P. aerug. ATCC 27853 | >8 | >8 | >4 | >4 | >4 | >4 | >4 | >4 | >8 | >8 | ./. | n.b. | 4 | 8 |
| ATCC BAA747 | 8 | >8 | 4 | >4 | 4 | >4 | 2 | 4 | 8 | >8 | 64 | 128 | 2 | 4 |

Tabelle 5b: Antimikrobielle Aktivität der ethanolischen Pflanzenextrakte-Kombinationen (1:1 Mischung)

| Testkeim | MIC | MBC | MIC | MBC | MIC | MBC | MIC | MBC | MIC | MBC | MIC | MBC |
|---|---|---|---|---|---|---|---|---|---|---|---|---|
| | A-E / B-E | | A-E / C-E | | B-E / C-E | | C-E / D1-E | | B-E / D1-E | | A-E / D1-E | |
| MRSA NCTC 10442 | 2/2 | >2/2 | >2/2 | >2/2 | 2/2 | >2/2 | 1/1 | 2/2 | 0,5/0,5 | 1/1 | 1/1 | 2/2 |
| S. aureus ATCC 25923 | 1/1 | >2/2 | 2/2 | >2/2 | 1/1 | >2/2 | 0,5/0,5 | 1/1 | 0,5/0,5 | 1/1 | 1/1 | 2/2 |
| S. epid. ATCC 14990 | 1/1 | 2/2 | 1/1 | >2/2 | 1/1 | 2/2 | 1/1 | 2/2 | 0,5/0,5 | 1/1 | 1/1 | 2/2 |

Tabelle 5c: Positivkontrollen (antimikro. Wirkung)

| Testkeim | MIC | MBC | MIC | MBC |
|---|---|---|---|---|
| | Van μg/ml | | Strep μg/ml | |
| MRSA NCTC 10442 | 1 | 2 | ./. | n.b. |
| S. aureus ATCC 25923 | 0,5 | 0,5 | 2 | 8 |
| S. epid. ATCC 14990 | 1 | 2 | 1 | 8 |
| | | | | |
| P. aerug. ATCC 27853 | ./. | n.b. | 4 | 8 |
| ATCC BAA747 | 64 | 128 | 2 | 4 |

Tabelle 5d: Zusammensetzung der Kontrollen

| Wachstumskontrolle | Medium + Testkeim ohne Extrakt/ohne Antibiotikum |
|---|---|
| Negativkontrolle | Medium + Testkeim + Lösungsmittel (Wasser/DMSO) ohne Extrakt/ohne Antibiotikum |
| Sterilitätskontrolle | Medium ohne weitere Zugaben |
| Positivkontrolle | Medium + Testkeim + Lösungsmittel (Wasser/DMSO) + Antibiotikum (siehe Tabelle 5c) |

**Beispiel 8a: Beispiele Formulierungen**

| Name | Homöopathische Verreibung | Tee | Tabletten & Pellets mit und ohne Filmüberzug | Hartgelatinekapseln | Weichgelatinekapseln | Lösung | Suspension | halbfeste Zubereitung | Puder | Füllstoff | Zerfallsbeschleuniger, Sprengmittel | Gleitmittel, Schmiermittel, Formtrennmittel, Fließregulierungsmittel | Lösungsmittel, Lösungsbeschleuniger | Emulgator, Lösungsvermittler, Solubilisator, Netzmittel, Antischaummittel | Salzbildner, Puffer | Gelbildner, Verdickungsmittel, Filmbildner, Bindemittel | Sorptionsmittel (Feuchthalte- bzw. Trockenmittel) | Süßmittel | Färbemittel | Weichmacher | Grundlage z.B. für Puder, Matrixbildner | Stabilisator |
|---|---|---|---|---|---|---|---|---|---|---|---|---|---|---|---|---|---|---|---|---|---|---|
| 1,2-Propylenglycol | | | 1 | 1 | 1 | | | | | | | | | | | | | | | 1 | | |
| Acetyltributylcitrat | | | 1 | 1 | 1 | | | | | | | | | | | | | | | 1 | | |
| Agar-Agar | | | | | 1 | | | | | | | | | | | 1 | | | | | | |
| Alkyl-4-hydroxybenzoate | | | 1 | 1 | 1 | 1 | 1 | | | | | | | | | | | | | | | 1 |
| Aluminium-Fettsäure-verbindungen | | | 1 | 1 | 1 | | | 1 | | | | | | | | | 1 | | | | | |
| Aluminium-oxid/-hydroxid | | | 1 | 1 | 1 | | | | | 1 | | | | | | | | | | | | 1 |
| Arabisches Gummi | | | | | 1 | | | | 1 | | | | | | | 1 | | | | | | |
| Ätherische Öle | 1 | 1 | 1 | 1 | 1 | 1 | 1 | 1 | 1 | | | | | | | | | | | 1 | | |
| Bentonit | | | | | | | 1 | 1 | | | | | | | | 1 | | | | | | |
| Calciumcarbonat | | | 1 | 1 | | | | | | 1 | | | | | 1 | | | | | | | |
| Calciumhydrogen-phosphat | 1 | | 1 | 1 | | | | 1 | 1 | 1 | | | | | | 1 | | | | | | |
| Carboxymethyl-cellulose-Natrium | | | 1 | 1 | 1 | 1 | 1 | 1 | | | | | | 1 | | 1 | | | | | | |
| Carotine | | | 1 | 1 | 1 | | | | | | | | | | | | | | 1 | | | |
| Cellulose | | | 1 | | 1 | | 1 | | | 1 | | | | | | | 1 | | | | | 1 |
| Celluloseacetat-phthalat | | | 1 | | 1 | | | | | | | | | | | 1 | | | | | | |
| Cetylalkohol | | | | | | | | 1 | | | | | | 1 | | | | | | | | |
| Cetylstearylalkohol | | | | | | | | 1 | | | | | | 1 | | | | | | | | |
| Citronensäure | | | 1 | | | 1 | 1 | | | | | | | | 1 | | | | | | | |
| Dextrose | | | 1 | | | 1 | 1 | | | 1 | | | | | | | | 1 | | | | |
| Dibutyl/Diethyl-phthalat | | | 1 | 1 | 1 | | | | | | | | | | | | | | | 1 | | |
| Dimethylpolysiloxane | | | | | | | 1 | 1 | | | | | | 1 | | | | | | | | |
| Eisenoxide | | | 1 | 1 | 1 | | | | | | | | | | | | | | 1 | | | |
| Ethylalkohol | 1 | | 1 | 1 | 1 | 1 | 1 | | | | | | 1 | | | | | | | | | |
| Ethylcellulose | | | 1 | | 1 | | | | | | | | | | | 1 | | | | | | 1 |
| Gelatine | | | 1 | 1 | | | | | | | | | | | | 1 | | | | | | 1 |
| Glyceroltriacetat | | | 1 | 1 | 1 | | | | | | | | | | | | | | | 1 | | |

| | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
| Glycin | | | | | 1 | | | 1 | | 1 | | | | | | |
| Glyerol | 1 | 1 | 1 | 1 | 1 | 1 | | | 1 | | | 1 | | 1 | | |
| Glyerolmonostearat | | 1 | | | | 1 | | | | 1 | | | | | | |
| hochdisperses Siliciumdioxid | 1 | 1 | 1 | | 1 | 1 | 1 | | 1 | | 1 | 1 | | | | 1 |
| Hydroxypropylmethyl-cellulose (HPMC) | | 1 | 1 | 1 | 1 | 1 | 1 | 1 | | | 1 | | | | 1 | |
| Hydroypropyl-methylcellulose-phthalat | | 1 | | 1 | | | | | | | 1 | | | | 1 | |
| Isopropylalkohol | 1 | 1 | 1 | 1 | 1 | 1 | | | 1 | | | | | | | |
| Isopropylmyristat | | | | | | 1 | | | 1 | | | | | | | |
| Lactose | 1 | 1 | | | | | | 1 | | | | | | | | |
| Lecithin | | | | 1 | | 1 | | | | 1 | | | | | | |
| Macrogol-1000-glycerol-monolaurat, -monostearat, -monooleat | | | | | | 1 | | | | 1 | | | | | | |
| Macrogol-1500-glyceroltriricinoleat | | | | | | 1 | | | | 1 | | | | | | |
| Macrogol-Gylerolhydroxy-stearat | | | | | | 1 | | | | 1 | | | | | | |
| Macrogolstearat 400 | | | | | | 1 | | | | 1 | | | | | | |
| Magnesiumstearat | | 1 | 1 | | | | | | 1 | | | | | | | |
| Maltodextrin | 1 | 1 | | | 1 | | | 1 | | | | | | | | 1 |
| Mannitol | | 1 | | | 1 | 1 | | 1 | | | | | 1 | 1 | | |
| Methylcellulose | | 1 | | | 1 | 1 | | | | | | | 1 | | | |
| Natriumcetylstearyl-sulfat | | 1 | | 1 | 1 | 1 | 1 | | | 1 | | | | | | |
| Natriumdioctylsulfo-succinat (auch K, Ca-Salze) | | | | | 1 | 1 | | | | 1 | | | | | | |
| Natriumdodecyl-sulfat | | 1 | | 1 | 1 | 1 | 1 | | | 1 | | | | | | |
| Natriumhydrogen-carbonat | | 1 | 1 | | 1 | | | 1 | | | 1 | | | | | |
| Ölsäureoleylester | | | | | | 1 | | | | 1 | | | | | | |
| Pektin | | | | 1 | 1 | 1 | | | | | | | 1 | | | |
| Poloxamer | | 1 | | 1 | 1 | 1 | | | | | 1 | | | | | |
| Polyacrylsäure | | | | 1 | 1 | | | | | | | | 1 | | | 1 |
| Polyethylenglycol | | 1 | 1 | 1 | 1 | 1 | 1 | 1 | 1 | 1 | 1 | | | 1 | 1 | |
| Polyethylenoxid | | 1 | 1 | 1 | 1 | 1 | 1 | 1 | 1 | 1 | 1 | | | 1 | | |
| Polymethacrylate | | 1 | 1 | 1 | | | | | | | 1 | | | 1 | | |
| Polyoxyl 23 laurylether, -20 cetostearylether, -10 oleylether | | | | | | 1 | | | | 1 | | | | | | |
| Polyoxyl 40 stearat, -50 stearat | | | | | | 1 | | | | 1 | | | | | | |
| Polysorbat 20, 60, 80, 40 | | | | | 1 | 1 | | | | 1 | | | | | | |
| Polyvinylacetat-Copolymere | | 1 | 1 | 1 | | | | | | | 1 | | | | | |
| Polyvinylalkohol | | 1 | 1 | 1 | 1 | 1 | | | | | 1 | | | | 1 | |
| Polyvinylpyrrolidon | | 1 | 1 | 1 | 1 | 1 | | 1 | | | 1 | | | | 1 | |
| Riboflavin | | 1 | 1 | 1 | 1 | 1 | | | | | | | | 1 | | |
| Rizinusöl | | 1 | 1 | 1 | | | | | | | | | 1 | | | |
| Saccharose | | 1 | | | | | | 1 | | | 1 | | 1 | | | |
| Sorbitanmonooleat, -palmitat, -stearat, -trioleat, -tristearat, -laurat | | | | | | 1 | | | | 1 | | | | | | |
| Sorbitol | | 1 | | 1 | | | | | | | | | 1 | 1 | 1 | |
| Stärke | | 1 | | | 1 | 1 | 1 | | | | 1 | | | | 1 | |
| Stärken, Reis-, Mais- Kartoffel-, Weizen- | | 1 | 1 | | 1 | 1 | | | | | | | | | | |
| Stearinsäure | | 1 | 1 | | | 1 | | | 1 | | 1 | | | | | |
| Stearylalkohol | | | | | | 1 | | | | 1 | | | | | | |
| Talkum | | 1 | 1 | | | 1 | | | 1 | | | | | | | 1 |
| Titandioxid | | 1 | 1 | | | 1 | 1 | | | | | | | 1 | | |
| Tragant | | 1 | | 1 | 1 | | | | | | 1 | | | | | |

|  |  |  |  |  |  |  |  |  |  |  |  |  |  |  |  |  |  |  |  |  |  |  |  |  |
|---|---|---|---|---|---|---|---|---|---|---|---|---|---|---|---|---|---|---|---|---|---|---|---|---|
| Triacetin | 1 | 1 | 1 |  |  |  |  |  |  |  |  | 1 |  |  |  |  |  |  |  |  |  | 1 |  |  |
| Triethanolamin |  |  |  | 1 |  |  |  |  |  |  |  |  |  | 1 |  |  |  |  |  |  |  |  |  |  |
| Triethylcitrat | 1 | 1 | 1 |  |  |  |  |  |  |  |  |  |  |  |  |  |  |  |  |  |  | 1 |  |  |
| Tromethamol |  |  |  | 1 |  |  |  |  |  |  |  |  |  | 1 |  |  |  |  |  |  |  |  |  |  |
| Vaselin |  |  |  |  | 1 |  |  |  |  |  |  |  |  |  |  |  |  |  |  |  |  |  |  | 1 |
| Weinsäure | 1 |  |  | 1 | 1 |  |  |  |  |  |  |  |  | 1 |  |  |  |  |  |  |  |  |  |  |
| Harnstoff (Urea) |  |  |  |  |  |  |  |  |  |  |  |  |  | 1 | 1 |  | 1 |  |  |  |  |  |  |  |
| Ceramide |  |  |  |  |  |  |  |  |  |  |  |  | 1 |  |  |  |  |  |  |  |  |  |  |  |
| Hyaluronsäure |  |  |  |  |  |  |  |  |  |  |  |  |  |  |  | 1 |  |  |  |  |  |  |  |  |

Retardierende Eigenschaften weisen z.B. die Hilfsstoffe Ethyl- und Methylcellulose, Hydroxypropylmethylcellulose, Celluloseacetatphthalat und Hydroypropylmethylcellulosephthalat auf.

**Beispiel 8b Rezepturen**

Grundrezeptur: Feuchtigkeits-spendende Creme

100 g Basiscreme DAC enthalten:

4,0 g Glycerolmonostearat 60
6,0 g Cetylalkohol
7,5 g Mittelkettige Triglyceride (Neutralöl, Miglyol 812)
25,5 g Weißes Vaselin
7,0 g Macrogol-20-glycerolmonostearat
10,0 g Propylenglycol
40,0 g gereinigtes Wasser

2. Rezeptur: Feuchtiakeits-spendende Creme mit B-E, A-E und D2

40 bis 97 g der Grundrezeptur

1 - 10 g Stachytarpheta jamaicensis (B-E)
1 - 10 g Bidens alba (A-E)
1 - 40 g Aloe vera (D2)
(Gesamtgewicht 100 g = 100 Gew.-%, jeweils bezogen auf das Trockengewicht)

3. Rezeptur: Feuchtigkeits-spendende Creme mit A-E, B-E, C-E und D2

30 bis 96 g der Grundrezeptur

1 - 10 g Bursera simaruba (C-E)
1 - 10 g Stachytarpheta jamaicensis (B-E)
1 - 10 g Bidens alba (A-E)
1 - 40 g Aloe vera (D2)
(Gesamtgewicht 100 g = 100 Gew.-%, jeweils bezogen auf das Trockengewicht)
**Grundtinktur D2a** (Gesamt: 100 Gew.-% bezogen auf das Trockengewicht): 70 Gew.-% eines Aloe-Extraktes (D2) wurden homogen gemischt in 100 ml 90-%-igem Ethanol
**Grundtinktur D2b** (Gesamt: 100 Gew.-% bezogen auf das Trockengewicht): 30 Gew.-% eines Aloe-Extraktes (D2) wurden homogen gemischt in
100 ml 90-%-igem Ethanol

4. Immunologisch aktive Tinkturen mit A-E, B-E, C-E und D2

Jeweils 100 ml Grundtinktur D2a oder D2b wurden gemischt mit:

**Tabelle 6**

| Pflanzenextrakt | Mischung | | | | | | | | | |
|---|---|---|---|---|---|---|---|---|---|---|
| | 44 (1:1:1) | 45 (1:1) | 46 (1:1) | 47 (1:1) | 48 (1:1:1) | 49 (1:1:1) | 50 (1:1:1) | 51 (1:1:3) | | 53 (1:3:1) |
| | | | | | | | | | | |
| Bid. alba (A-E) | 1 | 1,5 | 1,5 | 2,5 | 2 | 5 | 7 | 0,5 | | 0,5/5 |
| Sta. jamaicen. (B-E) | 1 | 0 | 1,5 | 0 | 2 | 5 | 7 | 0,5 | | 1,5/15 |
| Bur. simaruba (C-E) | 1 | 1,5 | 0 | 2,5 | 2 | 5 | 7 | 1,5 | | 0,5/5 |

Die Gew.-% Angaben in Tabelle 6 beziehen sich auf das Trockengewicht der Extrakte, welche gemäß Beispiel 3 oder 4, vorzugsweise gemäß Beispiel 4, erhalten werden. Der pH-Wert der vorgenannten Tinkturen 44, bis 53 wurde abhängig von der gewünschten Indikation der Haut eingestellt auf:

a) Tinktur für die Haut: pH 4,5 bis 5,5
b) Tinktur für die Mundschleimhaut: pH 6,7 bis 7,2
c) Tinktur für das Auge: pH 7,0 bis 7,5
d) Tinktur für Nasenschleimhaut: pH 5,5 bis 6,5
e) Tinktur für Ohren: pH 5,5 bis 6,1

Die vorgenannten Mischungen und Gewichts-%-Verhältnisse (Tabelle 6) können abhängig von der Indikation variieren. Ist zum Beispiel eine verstärkt antimikrobielle Wirkung erwünscht, z.B. bei bakteriellen Infektion der (Schleim-)Haut oder im Mund (Zahnfleisch), ist es erfindungsgemäß vorteilhaft die Extrakte (bzw. Trockenextrakte) mit den stärksten antimikrobiellen Wirkungen (Tabelle 5a/5b) zu kombinieren oder nur einen Extrakt, z.B. A-E, zu verwenden (z.B. Mischung 53). Bei starken Entzündungen z.B. Auge oder Ohr, kann es dagegen erfindungsgemäß vorteilhaft sein, den Gehalt der besonders anti-entzündlich wirksamen Extrakte zu erhöhen, z.B. A-E und/oder C-E (Tabelle 2 und 3). Hier wäre z.B. die Mischung 45 oder die Mischung 45 mit höheren Gew.-% Anteilen von jeweils 5-20 Gew.-% des jeweiligen Trockenextraktes denkbar. Bei gleichzeitiger antimikrobieller und antientzündlicher Indikation ist abhängig von dem Therapieziel die geeignete Kombination gemäß der Erfindung zu wählen.

**Beispiel 9: Anwendungen auf der Haut**

| Patient (w/m) | Symptom Erkrankung | Phyto-Gemisch | Dosierung | Applikation | Verlauf |
|---|---|---|---|---|---|
| 4 (w) Stand der Technik | Lymphödem (re oder li), Oberarm tlw. Unterarm ca. 2 cm mehr im Durchmesser | B + C + Minze (Geschm.) | 5 Becher tgl. frisch, stark, sehr grün, | Tee, warm, intern | 5 bis 6 Stunden unter Beobachtung, Nach 2 bis 3 Stunden symptomatische Verbesserung nach zwei Tagen: Rückbildung, Spannungsnachlass, kein Lymphödem mehr |
| 2b (m) | Trockene Haut, Pruritus, Kratzspuren, urtikarielles Ekzem, links, v.a. Unterarm, Ellenbeuge. | A+B+C+D2 (Grundtinktur D2a+44) | Von ca. 200 mg in 5 Tagen maximal 50 ml verbraucht, grob | Tinktur ca. 1-4 /Tag aufgetragen, 2 Tage → noch x 3 Tag. Danach tlw. leicht eingecremt (tr. Haut). | Am selben Tag Erleichterung. Nach 2 Tagen nur noch Kratzspuren (Krusten) übrig, aber trockene Haut. Ab 5. Tag noch winzige Reste Kratzspuren, aber trockene Haut. |

(fortgesetzt)

| Patient (w/m) | Symptom Erkrankung | Phyto-Gemisch | Dosierung | Applikation | Verlauf |
|---|---|---|---|---|---|
| 1d | Lippenherpes, Fläche ca.6 x 4 mm, nach 3-4 Stunden bereits große Schwellung, Blase | A+B+C+D2 (Grundtinktur D2a+51) | Tropfenweise 3-4 /Tag | Tinktur tropfenweise 3-4 /Tag auf den Lippenherpes aufgetragen | Zuerst keine Vergrößerung mehr, dann schnelles Abklingen der Symptome und der Schwellung; Austrocknung innerhalb von 2 Tagen; keine Entzündung mehr; Kleine Kruste, die durch Feuchtwundbehandlung mit Tinktur und Zinksalbe nach einigen Tagen abheilte ⇒ Aciclovir (Salbe wirkte schlechter |
| 1e | Verucöse Papillome an 3 Stellen, 5-12 mm groß, dermatologisch per Erygenum-Laser entfernt; nach 2 Tagen alle 3 entzündet; Tag 3: Pflaster-wechsel und Aufsprühen von Hautdesinfektion; Tag 4: Entzündung verstärkt; eitrige Sekretbildung | A+B+C+D2 (Grundtinktur D2a+51) | Tropfenweise 1-2/Tag | Tropfenweise ca. 1-2/Tag ab Tag 5 die betroffenen Stellen gut mit Tinktur benetzt | Tag 5: Einleitung der Behandlung wegen Entzündung und Verklebung mit dem Pflaster, Begonnene Sekretion an Tag 5; Die Entzündungsschmerzen ließen schnell nach. An Tag 2 der Anwendung erste Krustenbildung, kaum Sekret; gelegentlich leichter Juckreiz; Tag 3 der Anwendung: keine Sekretion mehr ⇒ schnell abheilend, Entzündung schnell weg |
| 18.1 | 4-5 entzündete Insektenstiche, 5-8 mm groß; dunkle Rötung, Schwellung, Juckreiz, Schmerzhaft; tlw. Sekret und Kratzspuren | A+B+C+D2 (Grundtinktur D2a+51) | Tropfenweise Mehrmals/Tag | Insektenstiche Mehrmals am Tag mit Tinktur benetzt | Juckreiz klang schnell ab; innerhalb von wenigen Stunden bis einem Tag keine Entzündung mehr; nur noch kleine Stellen ohne Schwellung sichtbar; am Tag 2 der Anwendung völlig abgeheilt |
| 19 (w) | 38-Jährige mit Brustkarzinom, familiär nicht prädisponiert; Neigung zu entzündeter Brust; u.a. Hautprobleme, Brust gerötet, verhärtet, schmerzhaft, entzündlich | A+B+C+D2 (Grundtinktur D2a+51) in Kombination mit einem Tee mit Bursera simaruba (C) | 2-3/Tag als Auflage | Ein Tuch wurde mit der Tinktur getränkt; Tuch als Auflage auf die Brust gelegt mit 30-60 min Einwirkzeit | Täglich verbesserter Zustand, Entzündung und Schmerzen ließen schnell nach Nach 1,5 Jahren nach Abschluss der Behandlung kein erneutes Auftreten der Brust-Entzündungen usw.; Brustkarzinom wurde entfernt (Verlauf nicht bekannt) |

**Patentansprüche**

1. Immunologisch aktives Phyto-Gemisch, welches eine antimikrobielle Wirksamkeit gegen eine transiente Hautflora und eine anti-entzündliche Wirksamkeit aufweist, wobei das Phyto-Gemisch mindestens einen ethanolischen Pflanzenextrakt ausgewählt aus

   a) Bidens alba (Bid. alba), Bidens pilosa (Bid. pilosa) aus dem Genus Bidens der Familie Asteraceae, und mindestens einen weiteren ethanolischen Pflanzenextrakt umfasst, ausgewählt aus
   b) Stachytarpheta jamaicensis (Sta. jamaicensis), Stachytarpheta cayennensis (Sta. cayennensis), Stachytarpheta indica (Sta. indica) aus dem Genus Stachytarpheta der Familie Verbenaceae und/oder
   c) Bursera simaruba (Bur. simaruba), Bursera microphylla (Bur. microphylla) und Bursera glabrifolia (Bur. glabrifolia) aus dem Genus Bursera der Familie Burseraceae.

2. Phyto-Gemisch nach Anspruch 1, **dadurch gekennzeichnet, dass** die transiente Hautflora umfasst Staphylococcen, Streptococcen, Methicillin-resistenten Staphylococcus aureus (MRSA), Pseudomonaden und/oder Acinetobakterien.

3. Phyto-Gemisch nach Anspruch 1 oder 2, **dadurch gekennzeichnet, dass** dieses eine antibakterielle Wirksamkeit umfasst bei einer Konzentration von größer gleich 10 $\mu$g/ml bis kleiner gleich 10 mg/ml, gemessen als minimale Hemmkonzentration (MHK/MIC) und/oder als minimale bakterizide Konzentration (MBK/MBC) der jeweiligen Extraktmischung.

4. Phyto-Gemisch nach einem der Ansprüche 1 bis 3, **dadurch gekennzeichnet, dass** dieses eine anti-entzündliche Wirksamkeit bei kleiner gleich 200 $\pm$ 10 $\mu$g/ml aufweist, gemessen als IC$_{50}$ der 5-LOX Inhibierung der jeweiligen Extraktmischung.

5. Phyto-Gemisch nach einem der Ansprüche 1 bis 4, **dadurch gekennzeichnet, dass** dieses vorliegt in

   - flüssiger Form umfassend Lösung, Dispersion, Suspension, Emulsion, Tinktur, Sirup, Saft und Tee
   - fester Form umfassend Tablette, Pulver, Puder, Dragee, Globuli, Granulat und Lyophylisat, Kapsel oder
   - als Gemisch umfassend Kapseln, Aerosol, Spray, Emulsion, Lotion und Creme.

6. Phyto-Gemisch nach einem der Ansprüche 1 bis 5, **dadurch gekennzeichnet, dass** das Phyto-Gemisch in flüssiger Form je nach Alternative einen für die Haut verträglichen pH größer gleich 3 bis kleiner gleich 9 aufweist, einen für die Mundschleimhaut verträglichen pH größer gleich 6 bis kleiner gleich 8, einen für die Nasenschleimhaut verträglichen pH größer gleich 5 bis kleiner gleich 7 oder einen für das Auge verträglichen pH größer gleich 7 bis kleiner gleich 9.

7. Phyto-Gemisch nach einem der Ansprüche 1 bis 6, **dadurch gekennzeichnet, dass** das flüssige Phyto-Gemisch als Tinktur vorliegt, umfassend

   - größer gleich 1 Gew.-%, bezogen auf den Gesamtgehalt der Tinktur (T = 100 Gew.-%), mindestens eines ethanolischen Pflanzenextraktes ausgewählt aus a) Bid. alba, Bid. pilosa, und mindestens einen weiteren ethanolischen Pflanzenextrakt ausgewählt aus b) Sta. jamaicensis, Sta. cayennensis, Sta. indica und/oder c) Bur. simaruba, Bur. microphylla, Bur. glabrifolia,
   - mindestens ein Säuerungsmittel umfassend Essigsäure, Citronensäure, Ascorbinsäure, Adipinsäure, Weinsäure, Mandelsäure, und/oder Apfelsäure,
   - größer gleich 1 Gew.-% eines Aloeextrakts, bezogen auf den Gesamtgehalt der Tinktur (T = 100 Gew.-%),

   wobei die Tinktur einen pH-Wert größer gleich 3 bis kleiner gleich 9 aufweist und die Tinktur ein wässrig/ethanolisches Gemisch ist mit einer Ethanolkonzentration größer gleich 70 %, bezogen auf die Gesamtzusammensetzung der Tinktur.

8. Verfahren zur Herstellung eines immunologisch aktiven Phyto-Gemisches nach einem der Ansprüche 1 bis 7 umfassend die Schritte

   - Bereitstellen mindestens eines Pflanzenbestandteils mindestens einer Pflanze ausgewählt aus a) und mindestens einer weiteren Pflanze ausgewählt aus b) und/oder c) jeweils ausgewählt aus ober- und/oder unterirdischen Pflanzenbestandteilen,
- jeweils mindestens einen Extraktionsschritt, wobei als Lösungsmittel wässriger Ethanol mit größer gleich 70 % Ethanol bis kleiner gleich 100 % Ethanol eingesetzt wird und
- Erhalten mindestens eines ethanolischen Pflanzenextraktes der jeweiligen Pflanze
- optional einen weiteren Verarbeitungsschritt umfassend Trocknen, Zerkleinerung, Mahlen mittels einer Mühle, Verarbeitung in einem Mörser, Dispergieren und
- Mischen mindestens zwei der ethanolischen Pflanzenextrakte, wobei einer ausgewählt wird aus a) und der mindestens zweite aus b) und/oder c) und
- optional Hinzumischen eines dritten Pflanzenextraktes ausgewählt aus b), c) und/oder d).

9. Verfahren nach Anspruch 8, wobei die mindestens zwei Pflanzenextrakte erhalten werden in flüssiger Form, in trockener Form oder als Gemisch aus festen und flüssigen Formen.

10. Verfahren nach Anspruch 8 oder 9, wobei die flüssige Form der mindestens zwei Pflanzenextrakte erhalten wird durch Trocknen der ethanolischen Extrakte und Zugabe eines wässrigen Lösungsmittels.

11. Immunologisch aktives Phyto-Gemisch nach einem der Ansprüche 1 bis 7 und/oder hergestellt nach einem der Ansprüche 8 bis 10 zur Verwendung als Arzneimittel, Medizinprodukt, Nahrungsergänzungsmittel, Kosmetikum und/oder als immunologisch aktiver Zusatz zu einem der vorgenannten Erzeugnisse.

12. Immunologisch aktives Phyto-Gemisch nach einem der Ansprüche 1 bis 7 oder 11, wobei das immunologisch aktive Phyto-Gemisch zur topischen und oralen Verabreichung in wässriger Form vorliegt.

13. Pharmazeutische oder kosmetische Zusammensetzung umfassend das immunologisch aktive Phyto-Gemisch nach einem der Ansprüche 1 bis 7, 11 oder 12.

14. Immunologisch aktives Phyto-Gemisch zur Anwendung bei der Prävention oder Behandlung von Effloreszenzen, welches eine antimikrobielle Wirksamkeit gegen eine transiente Hautflora und eine anti-entzündliche Wirksamkeit aufweist und wobei das Phyto-Gemisch mindestens einen ethanolischen Pflanzenextrakt enthält, ausgewählt aus

a) Bid. alba, Bid. pilosa der Familie Asteraceae, und mindestens einen weiteren ethanolischen Pflanzenextrakt umfasst, ausgewählt aus
b) Sta. jamaicensis, Sta. cayennensis, Sta. indica der Familie Verbenaceae und/oder
c) Bur. simaruba, Bur. microphylla und/oder Bur. glabrifolia der Familie Burseraceae. und optional d) mindestens einen weiteren Extrakt einer weiteren biologisch aktiven Pflanze.

15. Phyto-Gemisch zur Anwendung nach Anspruch 14, **dadurch gekennzeichnet, dass** Effloreszenzen umfassend kosmetische und pathologische Effloreszenzen, mit der transiente Hautflora assoziierte Hauterkrankungen, bakterielle und/oder virale Infektionen der Haut, Furunkulosen, Mykosen, Entzündungsreaktionen der Haut, Impetigo, benigne und maligne Tumorbildung, Dermatosen, Ekzeme, Pruritus, Psoriasis, Akne, Hautreizungen, Hautrötung, symptomatische Effloreszenzen, Verbrennungen, Verätzungen, Erfrierungen, durch Gifte, Arzneimittel, Drogen, Allergene, Strahlung und als Nebenwirkung auftretende Effloreszenzen, durch Bisse und Stiche von Insekten und Parasiten verursachte Reizungen und Entzündungen der Haut.

16. Phyto-Gemisch zur Anwendung nach Anspruch 14 oder 15, **dadurch gekennzeichnet, dass** das Phyto-Gemisch vorliegt als

- orale Formulierung umfassend i) feste Formen, wie Tablette, Pulver, Granulat, Brausetablette, Trockensaft, Dragee, Globuli, Kapsel und Lyophylisat, ii) flüssige Formen, wie Suspension, Lösung, Dispersion, Tinktur, Konzentrat, Tee, oder iii) Gemisch, wie Spray, Aerosol oder
- topische Formulierung umfassend i) feste Formen, wie Pulver, Badezusatz, Sitzbadpulver, ii) flüssige Formen, wie Suspension, Emulsion, Lösung, Dispersion, Tinktur, Tee oder iii) Gemisch, wie Spray, Aerosol, Lotionen, halbfeste Formen umfassend Salben, Cremes und Pasten.

17. Phyto-Gemisch zur Anwendung nach einem der Ansprüche 14 bis 16, **dadurch gekennzeichnet, dass** das Phyto-Gemisch als Tinktur vorliegt, umfassend

- größer gleich 1 Gew.-%, bezogen auf den Gesamtgehalt (T = 100 Gew.-%) der Tinktur, mindestens eines ethanolischen Pflanzenextraktes ausgewählt aus Bid. alba, Bid. pilosa und mindestens einen weiteren ethanolischen Pflanzenextrakt ausgewählt aus Sta. jamaicensis, Sta. cayennensis, Sta. indica und/oder Bur. simaruba,
- mindestens ein Säuerungsmittel ausgewählt aus Essigsäure, Citronensäure, Ascorbinsäure, Adipinsäure, Weinsäure, Mandelsäure und/oder Apfelsäure,
- größer gleich 1 Gew.-% eines Aloeextrakts, bezogen auf den Gesamtgehalt der Tinktur (T = 100 Gew.-%), und
- mindestens einen Hilfsstoff,

wobei die Tinktur einen pH-Wert größer gleich 3 bis kleiner gleich 9 aufweist und die Tinktur ein wässrig/ethanolisches Gemisch ist, mit einer Ethanolkonzentration größer gleich 70 %, bezogen auf die Gesamtzusammensetzung der Tinktur.

18. Phyto-Gemisch zur Anwendung nach einem der Ansprüche 14 bis 17, **dadurch gekennzeichnet, dass** die orale Formulierung auf einer festen Form basiert, wobei

- die feste Form mindestens einen getrockneten ethanolischen Pflanzenextrakt umfasst, ausgewählt aus Bid. alba, Bid. pilosa und mindestens einen weiteren getrockneten ethanolischen Pflanzenextrakt ausgewählt aus Sta. jamaicensis, Sta. cayennensis, Sta. indica und/oder Bur. simaruba und optional Aloe,
- der mindestens eine Pflanzentrakt mit einem Gehalt größer gleich 1 Gew.-%, bezogen auf den Gesamtgehalt der festen Form (F = 100 Gew.-%), enthalten ist,
- die Restfeuchte kleiner gleich 5 Gew.-% beträgt, bezogen auf den Gesamtgehalt des trockenen Pflanzenextrakts (F = 100 Gew.-%) und
- mindestens ein Hilfsstoff enthalten ist.

**Claims**

1. Immunologically active phyto-mixture exhibiting an antimicrobial efficiency against transient skin flora and an anti-inflammatory efficiency, the phyto-mixture comprising at least one ethanolic plant extract selected from

a) Bidens alba (Bid. alba), Bidens pilosa (Bid. pilosa) from genus Bidens of the Asteraceae family, and comprises at least one further ethanolic plant extract selected from
b) Stachytarpheta jamaicensis (Sta. jamaicensis), Stachytarpheta cayennensis (Sta. cayennensis), Stachytarpheta indica (Sta. indica) from genus Stachytarpheta of the Verbenaceae family, and/or
c) Bursera simaruba (Bur. simaruba), Bursera microphylla (Bur. microphylla) and Bursera glabrifolia (Bur. glabrifolia) from genus Bursera of the Burseraceae family.

2. Phyto-mixture according to claim 1, **characterised in that** the transient skin flora comprises staphylococci, streptococci, methicillin-resistant Staphylococcus aureus (MRSA), pseudomonads and/or acinetobacteria.

3. Phyto-mixture according to claim 1 or 2, **characterised in** comprising an antibacterial efficiency at a concentration of greater than or equal to 10 $\mu$g/ml to less than or equal to 10 mg/ml measured as minimal inhibitory concentration (MHK/MIC) and/or as minimum bactericidal concentration (MBK/MBC) of the respective extract mixture.

4. Phyto-mixture according to any one of the claims 1 to 3, **characterised in** exhibiting an anti-inflammatory efficiency at less than or equal to 200 $\pm$ 10 $\mu$g/ml measured as IC$_{50}$ of 5-LOX inhibition of the respective extract mixture.

5. Phyto-mixture according to any one of the claims 1 to 4, **characterised in** being present in

- liquid form comprising solution, dispersion, suspension, emulsion, tincture, syrup, juice, and tea
- solid form comprising tablet, powder, dragee, globules, granules and lyophilisate, capsule, or
- as mixture comprising capsules, aerosol, spray, emulsion, lotion, and cream.

6. Phyto-mixture according to any one of the claims 1 to 5, **characterised in that** the phyto-mixture in liquid form, depending on the alternative, has a pH tolerated by the skin greater than or equal to 3 to less than or equal to 9, a pH tolerated by the oral mucosa greater than or equal to 6 to less than or equal to 8, a pH tolerated by the nasal mucosa greater than or equal to 5 to less than or equal to 7, or a pH tolerated by the eye greater than or equal to 7 to less than or equal to 9.

**7.** Phyto-mixture according to any one of the claims 1 to 6, **characterised in that** the liquid phyto-mixture is present as tincture, comprising

- greater than or equal to 1 % by weight, based on the total content of the tincture (T = 100 % by weight), of at least one ethanolic plant extract selected from a) Bid. alba, Bid. pilosa, and at least one further ethanolic plant extract selected from b) Sta. jamaicensis, Sta. cayennensis, Sta. indica, and/or c) Bur. simaruba, Bur. microphylla, Bur. glabrifolia,
- at least one acidifier comprising acetic acid, citric acid, ascorbic acid, adipic acid, tartaric acid, mandelic acid, and/or malic acid,
- greater than or equal to 1 % by weight of an aloe extract, based on the total content of the tincture (T = 100 % by weight),

the tincture having a pH value of greater than or equal to 3 to less than or equal to 9 and the tincture is an aqueous/ethanolic mixture having an ethanol concentration greater than or equal to 70 %, based on the total composition of the tincture.

**8.** Method for the production of an immunologically active phyto-mixture according to any one of the claims 1 to 7 comprising the steps of

- providing at least one plant part of at least one plant selected from a) and at least one further plant selected from b) and/or c), each selected from above- and/or underground plant parts,
- respectively at least one extraction step using as solvent aqueous ethanol with greater than or equal to 70 % ethanol to less than or equal to 100 % ethanol, and
- obtaining at least one ethanolic plant extract of the respective plant,
- optionally, a further processing step comprising drying, crushing, milling by means of a mill, processing in a mortar, dispersing, and
- mixing at least two of the ethanolic plant extracts, one being selected from a) and the at least one second being selected from b) and/or c), and
- optionally, adding a third plant extract selected from b), c) and/or d).

**9.** Method according to claim 8, wherein the at least two plant extracts are obtained in liquid form, in dry form or as mixture of solid and liquid forms.

**10.** Method according to claims 8 or 9, wherein the liquid form of the at least two plant extracts is obtained by drying the ethanolic extracts and adding an aqueous solvent.

**11.** Immunologically active phyto-mixture according to any one of the claims 1 to 7 and/or produced according to any one of the claims 8 to 10 for use as medicament, medical product, nutritional supplement, cosmetic, and/or as an immunologically active addition to one of the afore-mentioned products.

**12.** Immunologically active phyto-mixture according to any one of the claims 1 to 7 or 11, wherein the immunologically active phyto-mixture is present in liquid form for oral and topical administration.

**13.** Pharmaceutic or cosmetic composition comprising the immunologically active phyto-mixture according to any one of the claims 1 to 7, 11 or 12.

**14.** Immunologically active-phyto mixture for use in the prevention or treatment of efflorescences exhibiting an antimicrobial efficiency against transient skin flora and an anti-inflammatory efficiency, and the phyto-mixture comprising at least one ethanolic plant extract selected from

a) Bid. alba, Bid. pilosa of the Asteraceae family, and at least one further ethanolic plant extract selected from
b) Sta. jamaicensis, Sta. cayennensis, Sta. indica of the Verbenaceae family, and/or
c) Bur. simaruba, Bur. microphylla and/or Bur. glabrifolia of the Burseraceae family and, optionally, d) at least one further extract of a biologically active plant..

**15.** Phyto-mixture for use according to claim 14, **characterised in that** efflorescences comprise cosmetic or pathologic efflorescences, skin diseases associated with the transient skin flora, bacterial and/or viral infections of the skin, furunculoses, mycoses, inflammatory reactions of the skin, impetigo, benign and malign tumor formation, dermatoses, eczemas, pruritus, psoriasis, acne, skin irritations, erythema, symptomatical efflorescences, burns, chemical burns, frostbites, efflorescences occurring by toxins, medicaments, drugs, allergens, radiation and efflorescences occurring as side effect, irritations and inflammations of the skin caused by bites and stings of insects and parasites.

**16.** Phyto-mixture for use according to claim 14 or 15, **characterised in that** the phyto-mixture is present as

- oral formulation comprising i) solid forms, such as tablet, powder, granules, effervescent tablet, dry syrup, dragee, globules, capsule and lyophilisate, ii) liquid forms, such as suspension, solution, dispersion, tincture, concentrate, tea, or iii) mixture, such as spray, aerosol, or
- topical formulation comprising i) solid forms, such as powder, bath additive, hip bath powder, ii) liquid forms, such as suspension, emulsion, solution, dispersion, tincture, tea, or iii) mixture, such as spray, aerosol, lotions, semi-solid forms comprising ointments, creams and pastes.

**17.** Phyto-mixture for use according to any one of the claims 14 to 16, **characterised in that** the phyto-mixture is present as tincture, comprising

- greater than or equal to 1 % by weight, based on the total content (T = 100 % by weight) of the tincture, of at least one ethanolic plant extract selected from Bid. alba, Bid. pilosa, Sta. jamaicensis, and at least one further ethanolic plant extract selected from Sta. cayennensis, Sta. indica and/or Bur. simaruba,
- at least one acidifier selected from acetic acid, citric acid, ascorbic acid, adipic acid, tartaric acid, mandelic acid and/or malic acid,
- greater than or equal to 1 % by weight of an Aloe extract, based on the total content of the tincture (T = 100 % by weight), and
- at least one excipient,

the tincture having a pH value greater than or equal to 3 to less than or equal to 9 and the tincture is an aqueous/ethanolic mixture, having an ethanol concentration greater than or equal to 70 %, based on the total composition of the tincture.

**18.** Phyto-mixture for use according to any one of the claims 14 to 17, **characterised in that** the oral formulation is based on a solid form,

- the solid form comprising at least one dried ethanolic plant extract, selected from Bid. alba, Bid. pilosa, and at least one further dried ethanolic plant extract selected from Sta. jamaicensis, Sta. cayennensis, Sta. indica and/or Bur. simaruba and, optionally, Aloe,
- the at least one plant extract being contained in a content of greater than or equal to 1 % by weight, based on the total content of the solid form (F = 100 % by weight),
- the residual moisture being less than or equal to 5 % by weight, based on the total content of the dry plant extract (F = 100 % by weight), and
- at least one excipient being contained.

## Revendications

**1.** Phyto-mélange immunologiquement actif montrant une efficacité antimicrobienne contre une flore cutanée transitoire et une efficacité anti-inflammatoire, le phyto-mélange comprenant au moins un extrait de plante éthanolique sélectionné à partir de

a) Bidens alba (Bid. alba), Bidens pilosa (Bid. pilosa) du genre Bidens de la famille des Asteraceae, et comprend au moins un autre extrait de plante éthanolique sélectionnée à partir de
b) Stachytarpheta jamaicensis (Sta. jamaicensis), Stachytarpheta cayennensis (Sta. cayennensis), Stachytarpheta indica (Sta. indica) du genre Stachytarpheta de la famille des Verbenaceae, et/ou
c) Bursera simaruba (Bur. simaruba), Bursera microphylla (Bur. microphylla) et Bursera glabrifolia (Bur. glabrifolia) du genre Bursera de la famille des Burseraceae.

**2.** Phyto-mélange selon la revendication 1, **caractérisé en ce que** la flore cutanée transitoire comprend les staphylocoques, les streptocoques, le Staphylococcus aureus résistant à la méticilline (MRSA), les pseudomonades et/ou les bactéries acineto.

3. Phyto-mélange selon la revendication 1 ou 2, caractérisé en comprenant une efficacité antibactérienne à une concentration de supérieure ou égale à 10 μg/ml jusqu'à inférieure ou égale à 10 mg/ml mesurée comme la concentration minimale inhibitrice (MHK/MIC) et/ou comme la concentration minimale bactéricide (MBK/MBC) du mélange d'extrait respectif.

4. Phyto-mélange selon l'une des revendications 1 à 3, caractérisé en montrant une efficacité anti-inflammatoire à inférieure ou égale à 200 ± 10 μg/ml mesurée comme la $CI_{50}$ de l'inhibition de 5-LOX du mélange d'extrait respectif.

5. Phyto-mélange selon l'une des revendications 1 à 4, caractérisé en étant présent sous

   - forme liquide comprenant une solution, une dispersion, une suspension, une émulsion, une teinture, un sirop, un jus, et un thé
   - forme solide comprenant un comprimé, une poudre, une dragée, des globules, des granulés et un lyophilisat, une capsule, ou
   - comme un mélange comprenant des capsules, un aérosol, un spray, une émulsion, une lotion, ou une crème.

6. Phyto-mélange selon l'une des revendications 1 à 5, **caractérisé en ce que** le phyto-mélange sous la forme liquide, dépendant de l'alternative, a un pH toléré par la peau supérieur ou égal à 3 jusqu'à inférieur ou égal à 9, un pH toléré par la muqueuse buccale supérieur ou égal à 6 jusqu'à inférieur ou égal à 8, un pH toléré par la muqueuse nasale supérieur ou égal à 5 jusqu'à inférieur ou égal à 7, ou un pH toléré par l'œil supérieur ou égal à 7 jusqu'à inférieur ou égal à 9.

7. Phyto-mélange selon l'une des revendications 1 à 6, **caractérisé en ce que** le phyto-mélange liquide est présent comme teinture, comprenant

   - supérieur ou égal à 1 % en poids, par rapport à la teneur totale de la teinture (T = 100 % en poids), d'au moins un extrait de plante éthanolique sélectionné à partir de a) Bid. alba, Bid. pilosa, et au moins un autre extrait de plante éthanolique sélectionné à partir de b) Sta. jamaicensis, Sta. cayennensis, Sta. indica, et/ou c) Bur. simaruba, Bur. microphylla, Bur. glabrifolia,
   - au moins un acidifiant comprenant de l'acide acétique, de l'acide citrique, de l'acide ascorbique, de l'acide adipique, de l'acide tartrique, de l'acide mandélique, et/ou de l'acide malique,
   - supérieur ou égal à 1 % en poids d'un extrait d'aloès, par rapport à la teneur totale de la teinture (T = 100 % en poids),

   la teinture ayant une valeur pH supérieure ou égale à 3 jusqu'à inférieure ou égale à 9 et la teinture est un mélange aqueux/éthanolique ayant une concentration d'éthanol supérieure ou égal à 70 %, par rapport à la composition totale de la teinture.

8. Procédé pour la production d'un phyto-mélange immunologiquement actif selon l'une des revendications 1 à 7, comprenant les étapes de

   - fournir au moins une partie de plante d'au moins une plante sélectionnée à partir de a) et au moins une autre plante sélectionnée de b) et/ou c), chacune sélectionnée des parties de plante à la surface et souterraines,
   - respectivement au moins une étape d'extraction utilisant comme solvant l'éthanol aqueux avec supérieur ou égal à 70 % éthanol jusqu'à inférieur ou égal à 100 % éthanol, et
   - obtenir au moins un extrait de plante éthanolique de la plante respective,
   - facultativement, une autre étape de traitement comprenant le séchage, le broyage, le moulage en moyen d'un moulin, le traitement dans un mortier, la dispersion, et
   - mélanger au moins deux des extraits de plante éthanoliques, l'un étant sélectionné à parti de a) et l'au moins deuxième à partir de b) et/ou c), et
   - facultativement, ajouter un troisième extrait de plante sélectionné à partir de b), c) et/ou d).

9. Procédé selon la revendication 8, selon lequel l'au moins deux extraits de plante sont obtenus sous forme liquide, sous forme sèche ou comme mélange des formes solides et liquides.

10. Procédé selon la revendication 8 ou 9, selon lequel la forme liquide des au moins deux extraits de plante est obtenue

EP 3 256 142 B1

par sécher les extraits éthanoliques et ajouter un solvant aqueux.

11. Phyto-mélange immunologiquement actif selon l'une des revendications 1 à 7 et/ou produit selon l'une des revendications 8 à 10 pour utilisation comme médicament, produit médical, complément nutritionnel, cosmétique, et/ou comme un ajout immunologiquement actif à l'un des produits susmentionnés.

12. Phyto-mélange immunologiquement active selon l'une des revendications 1 à 7 ou 11, selon lequel le phyto-mélange est présent sous forme liquide pour l'administration orale et topique.

13. Composition pharmaceutique ou cosmétique comprenant le phyto-mélange immunologiquement actif selon l'une des revendications 1 à 7, 11 ou 12.

14. Phyto-mélange immunologiquement actif pour utilisation dans la prévention et le traitement des efflorescences montrant une efficacité antimicrobienne contre une flore cutanée transitoire et une efficacité anti-inflammatoire, et le phyto-mélange comprenant au moins un extrait de plante éthanolique sélectionné à partir de

a) Bid. alba, Bid. pilosa de la famille des Asteraceae, et au moins un autre extrait de plante éthanolique sélectionné à partir de
b) Sta. jamaicensis, Sta. cayennensis, Sta. indica de la famille des Verbenaceae, et/ou
c) Bur. simaruba, Bur. microphylla et/ou Bur. glabrifolia de la famille des Burseraceae et, facultativemenet, d) au moins un autre extrait d'une autre plante biologiquement active.

15. Phyto-mélange pour utilisation selon la revendication 14, **caractérisé en ce que** les efflorescences comprennent des efflorescences cosmétiques ou pathologiques, des maladies cutanées associées avec la flore cutanée transitoire, des infections bactériennes et/ou virales de la peau, des furonculoses, des mycoses, des réactions inflammatoires de la peau, l'impétigo, une formation de tumeur bénigne et maligne, des dermatoses, des eczémas, le prurit, le psoriasis, l'acné, des irritations cutanées, l'érythème, des efflorescences symptomatiques, des brûlures, des brûlures chimique, des gelures, des efflorescences survenant par des toxines, des médicaments, des drogues, des allergènes, la radiation et des efflorescences survenant comme effet secondaire, des irritations et des inflammations de la peau causé par des morsures et des piqûres des insectes et des parasites.

16. Phyto-mélange pour utilisation selon la revendication 14 ou 15, **caractérisé en ce que** le phyto-mélange est présent comme

- formulation orale comprenant i) des formes solides, telles qu'un comprimé, une poudre, des granulés, un comprimé effervescent, un sirop sec, une dragée, des globules, une capsule et un lyophilisat, ii) des formes liquides, telles qu'une suspension, une solution, une dispersion, une teinture, un concentré, un thé, ou iii) des formes liquides, telles qu'un spray, un aérosol, ou
- formulation topique comprenant i) des formes solides, telles qu'une poudre, un ajout de bain, une poudre du bain de siège, ii) des formes liquides, telles qu'une suspension, une émulsion, une solution, une dispersion, une teinture, un thé, ou iii) un mélange, tel qu'un spray, un aérosol, des lotions, des formes semi-solides comprenant des onguents, des crèmes et des pâtes.

17. Phyto-mélange pour utilisation selon l'une des revendications 14 à 16, **caractérisée en ce que** le phyto-mélange est présent comme teinture, comprenant

- supérieur ou égal à 1 % en poids, par rapport à la teneur totale de la teinture (T = 100 % en poids), d'au moins un extrait de plante éthanolique sélectionné à partir de Bid. alba, Bid. pilosa, et au moins un autre extrait de plante éthanolique sélectionné à partir de Sta. jamaicensis, Sta. cayennensis, Sta. indica, et/ou Bur. simaruba,
- au moins un acidifiant comprenant de l'acide acétique, de l'acide citrique, de l'acide ascorbique, de l'acide adipique, de l'acide tartrique, de l'acide mandélique, et/ou de l'acide malique,
- supérieur ou égal à 1 % en poids d'un extrait d'aloès, par rapport à la teneur totale de la teinture (T = 100 % en poids), et
- au moins un excipient,

la teinture ayant une valeur pH supérieure ou égale à 3 jusqu'à inférieure ou égale à 9 et la teinture est un mélange aqueux/éthanolique ayant une concentration d'éthanol supérieure ou égal à 70 %, par rapport à la composition totale de la teinture.

**18.** Phyto-mélange pour utilisation selon l'une des revendications 14 à 17, **caractérisé en ce que** la formulation orale est fondée sur une forme solide

- la forme solide comprenant au moins un extrait de plante éthanolique séché, sélectionné de Bid. alba, Bid. pilosa, et au moins un autre extrait de plant éthanolique séché sélectionné de Sta. jamaicensis, Sta. cayennensis, Sta. indica et/ou Bur. simaruba et, facultativement, Aloe,
- l'au moins un extrait de plante étant contenu dans une teneur de supérieure ou égale à 1 % en poids, par rapport à la teneur totale de la forme solide (F = 100 % en poids),
- l'humidité résiduelle étant inférieure ou égale à 5 % en poids, par rapport à la teneur totale de l'extrait de plante séché (F = 100 % en poids), et
- au moins un excipient étant contenu.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- RU 2412719
- CN 102743651
- EP 114709 A1
- JP 2001178390 A

**In der Beschreibung aufgeführte Nicht-Patentliteratur**

- Screening for antimicrobial activity of ten medicinal plants used in Colombian folkloric medicine: A possible alternative in the treatment of non-nosocomial infections. **ROJAS JHON J.** BMC COMPLEMENTARY AND ALTERNATIVE MEDICINE. BIOMED CENTRAL LTD, 17. Februar 2006, vol. 6, 2
- **MANTARING-CHUA NIMFA.** Quantitative microbial assay, clinical testing and stability studies of the crude leaf extract of Bidens pilosa LINN. *ACTA MAN1LANA. SERIES A, NATURAL AND APPLIED SCIENCES,* 01. Januar 1991, vol. 39, 31-37
- Screening of medicinal plants from Trinidad and Tobago for antimicrobial and insecticidal properties. **CHARIANDY C M.** JOURNAL OF ETHNOPHARMACOLOGY. ELSEVIER IRELAND LTD, 21. Juli 1998, vol. 64, 265-270
- **OKOYE TC et al.** *Antimicrobial and antispasmodic activity of leaf extract and fractions of Stachytarpheta cayennensis,* 01. Januar 2010, 189-192
- **CAMPORESE A. et al.** Screening of anti-bacterial activity of medicinal plants from Belize (Central America). *JOURNAL OF ETHNOPHARMACOLOGY,* 01. Juli 2003, vol. 87 (1), ISSN 0378-8741, 103-107
- Topical Antibiotics in Dermatology: Emerging Patterns of Resistance. **ELSTON D. M.** DERMATOLOGIC CLINICS. W.B. SAUNDERS CO, 01. Januar 2009, vol. 27, 25-31